# EUROPÄISCHE PATENTSCHRIFT

(11) **EP 3 629 887 B1**
(45) Veröffentlichungstag und Bekanntmachung des Hinweises auf die Patenterteilung: **06.07.2022**
(21) Anmeldenummer: 18728120.9
(22) Anmeldetag: 29.05.2018
(51) Int. Cl.: A61B 5/00

(54) **HAARZUSTANDS-ERMITTLUNGSVORRICHTUNG, HAARZUSTANDS-ERMITTLUNGSSYSTEM UND VERFAHREN ZUM ERMITTELN EINES HAARZUSTANDS**
HAIR CONDITION-DETERMINING DEVICE, HAIR CONDITION-DETERMINING SYSTEM, AND METHOD FOR DETERMINING A HAIR CONDITION
DISPOSITIF DE DÉTERMINATION DE L'ÉTAT DES CHEVEUX, SYSTÈME DE DÉTERMINATION DE L'ÉTAT DES CHEVEUX ET PROCÉDÉ POUR LA DÉTERMINATION D'UN ÉTAT DES CHEVEUX

(30) Priorität: 31.05.2017 DE 102017209225
(43) Veröffentlichungstag der Anmeldung: 08.04.2020
(73) Patentinhaber: Henkel AG & Co. KGaA, 40589 Düsseldorf (DE)
(72) Erfinder: BLANK, Philippe, 47533 Kleve (DE)
(86) Internationale Anmeldenummer: PCT/EP2018/063984
(87) Internationale Veröffentlichungsnummer: WO 2018/219892

(56) Entgegenhaltungen:
- WO-A1-2017/032636
- WO-A2-02/24071
- US-A1- 2011 155 161
- US-A1- 2012 320 191
- US-A1- 2015 342 515

## Beschreibung

Die Erfindung betrifft eine Haarzustands-Ermittlungsvorrichtung, ein Haarzustands-Ermittlungssystem und ein Verfahren zum Ermitteln eines Haarzustands.

In vielen Bereichen des täglichen Lebens gibt es seit einiger Zeit einen Trend zu personalisierten Programmen, die auf individuelle Voraussetzungen und Bedürfnisse gezielt eingehen können, beispielsweise in einem Ernährungs- oder Gesundheitsbereich, aber auch in einem Bereich personalisierter Kosmetik. Diese kann es einem Nutzer ermöglichen, gezielt Kosmetikprodukte zu finden und/oder Pflegehinweise zu erhalten, die auf individuelle Bedürfnisse seiner Haare abgestimmt sind, und somit eine besonders hohe Wirksamkeit ermöglichen.

Bei einer Behandlung von Haar mit kosmetischen Produkten kann eine Wirkung des Produkts, z.B. eine Intensität einer Färbung, eine Wirksamkeit eines Pflegeprodukts oder eine Haarumformungswirkung einer Dauerwelle, stark von einem Haarzustand, insbesondere einem Schädigungsgrad des Haars abhängen.

Deshalb kann eine Ermittlung einer Schädigung des Haars von großer Bedeutung sein und für den Nutzer einen wichtigen Parameter zur (objektiven) Beurteilung seiner Haargesundheit darstellen.

Die Schädigung des Haars kann durch natürliche oder künstlich herbeigeführte Vorgänge passieren. Der wichtigste Schädigungstyp kann dabei eine oxidative Schädigung sein.

Die natürlichen Vorgänge können beispielsweise eine kombinierte (z.B. gleichzeitige) Einwirkung von UV-Licht und Sauerstoff (O₂) auf das Haar aufweisen.

Die künstlich herbeigeführten Vorgänge können dabei beispielsweise ein Anwenden von Haarfärbemitteln (auch als Colorationen bezeichnet, wozu hierin auch ein Blondieren gezählt wird), und/oder ein Stylen oder Umformen des Haars (z.B. ein Erzeugen einer Dauerwelle) aufweisen.

Dabei kann neben erwünschten kosmetischen Effekten, wie z.B. einer Aufhellung des Haars, auch eine starke Schädigung des Haars auftreten, beispielsweise bei einer Verwendung von Oxidationsmitteln.

Bei geschädigtem Haar kann beispielsweise ein Cysteinsäuregehalt erhöht sein wegen einer Oxidation der im Haar sehr häufig vorkommenden Aminosäuren Cystin und Cystein zu Cysteinsäure.

Dabei kann neben erwünschten kosmetischen Effekten, wie z.B. einer Aufhellung des Haars, auch eine starke Schädigung des Haars auftreten, beispielsweise bei einer Verwendung von Oxidationsmitteln.

Bei geschädigtem Haar kann beispielsweise ein Cysteinsäuregehalt erhöht sein wegen einer Oxidation der im Haar sehr häufig vorkommenden Aminosäuren Cystin und Cystein zu Cysteinsäure.

Die Oxidation des Cystins/Cysteins zu Cysteinsäure kann die mechanische Stabilität des Haars zerstören und bei mehrfachen Anwendungen sogar zu einem vollständigen Haarbruch führen. Allerdings können bereits vorher makroskopisch wahrnehmbare, z.B. fühlbare, Eigenschaften des Haars, beispielsweise eine Oberflächenbeschaffenheit, z.B. eine Oberflächenrauhigkeit, negativ beeinflusst werden. Geschädigtes Haar kann beispielsweise eine höhere Oberflächenrauhigkeit aufweisen als ungeschädigtes Haar.

Das menschliche Haar besteht aus Proteinen, Lipiden, Wasser, Spurenelementen und Pigmenten. Das Faserprotein Keratin ist der Hauptbestandteil der Haare. Die Lipide, die im menschlichen Haar vorhanden sind, sind entweder frei oder kovalent gebunden. Melanine sind rötliche, braune oder schwarze Pigmente, die die Färbung der Haare bewirken.

Ergebnisse kosmetischer Behandlungen können von weiteren Eigenschaften des behandelten Haars abhängen, beispielsweise (insbesondere bei einer Coloration) von einer Haarfarbe, von einer Haarstruktur (insbesondere bei einem Styling, z.B. einer Dauerwelle, einer Glättung usw.), von einem Feuchtigkeitsgehalt (bei einem Pflegeprodukt), usw.

Dokument WO 2017/032636 A1 beschreibt ein bildgebendes Gerät, das die Eigenschaften von Haaren durch die Analyse vergrößerter Bilder von Haaren bestimmt. Dokument US 2015/0342515

A1 beschreibt eine Bürste, die einen Beschleunigungssensor und ein Bilderfassungsgerät zur Bestimmung von Haareigenschaften enthält.

Gemäß der vorliegenden Erfindung wird eine Haarzustands-Ermittlungsvorrichtung bereitgestellt, welche es ermöglicht, mittels eines optischen Sensors einen Haarzustand eines Nutzers zu ermitteln.

Die vorliegende Erfindung betrifft eine Haarzustands-Ermittlungsvorrichtung zum Bereitstellen einer Information bezüglich eines Haarzustands von Haaren eines Nutzers , aufweisend:einen Vorrichtungskörper mit mindestens einem ersten Bereich und einem zweiten Bereich, welche so gestaltet sind, dass die Haare des Nutzers zwischen dem ersten Bereich und dem zweiten Bereich bewegbar sind;mindestens eine im oder am Vorrichtungskörper angeordnete optionale Lichtquelle zum Beleuchten der Haare des Nutzers;mindestens einen im oder am Vorrichtungskörper angeordneten optischen Sensor zum Erfassen von Licht, welches von der Lichtquelle abgestrahlt wurde und mit den Haaren gewechselwirkt hat, und/oder von Umgebungslicht, welches mit den Haaren gewechselwirkt hat; undeine im oder am Vorrichtungskörper angeordnete elektronische Schaltkreisvorrichtung,wobei die elektronische Schaltkreisvorrichtung mit dem optischen Sensor gekoppelt ist zum Empfangen eines mittels des erfassten Lichts erzeugten Signals, undwobei die elektronische Schaltkreisvorrichtung eingerichtet ist, basierend auf dem empfangenen Signal eine Information bezüglich des Haarzustands des Nutzers zu ermitteln und dem Nutzer die Information bereitzustellen,wobei die Haarzustands-Ermittlungsvorrichtung so eingerichtet ist, dass mittels des optischen Sensors ein Ermitteln eines Grads der Haarschädigung durch eine Bestimmung eines Gehaltes an Cysteinsäure ermittelt wird, wobei der optische Sensor eingerichtet ist, eine oder mehrere Aufnahmen in einem Fluoreszenzbereich und/oder in einem Nahinfrarotbereich zu machen,wobei die Haarzustands-Ermittlungsvorrichtung ferner so eingerichtet ist, dass mittels des optischen Sensors ein Wassergehalt des Haars ermittelt wird.

Die vorliegender Erfindung betrifft ebenfalls ein Haarzustands-Ermittlungssystem, aufweisend: die erfindungsgemäße Haarzustands-Ermittlungsvorrichtung; und eine Anzeigevorrichtung, wobei die mindestens eine Haarzustands-Ermittlungsvorrichtung eingerichtet ist, der Anzeigevorrichtung die Haarzustandsinformation mittels der Datenaustauschvorrichtung zu übermitteln.

Darüber hinaus betrifft die vorliegende Erfindung ein Verfahren zum Bereitstellen einer Haarzustandsinformation bezüglich Haaren eines Nutzers, aufweisend: Anordnen der erfindungsgemäßen Haarzustands-Ermittlungsvorrichtung oder des erfindungsgemäßen Haarzustands-Ermittlungssystems derart, dass die Haare des Nutzers einem Lichteintrittsbereich der Haarzustands-Ermittlungsvorrichtung zugewandt sind; optionales Beleuchten des Haars mittels der Lichtquelle; gegebenenfalls während des Beleuchtens, Erfassen zumindest eines Teils des Lichts, welches mit den Haaren gewechselwirkt hat, und/oder von Umgebungslicht, welches mit den Haaren gewechselwirkt hat; Ermitteln einer Haarzustandsinformation bezüglich der Haare des Nutzers basierend auf dem erfassten Licht; und Bereitstellen der Haarzustandsinformation.

Das Ermitteln des Haarzustands kann in verschiedenen Ausführungsbeispielen während eines Bürstens oder Kämmens des Haars erfolgen, d.h. die Haarzustands-Ermittlungsvorrichtung kann Kamm- oder Bürstenartig geformt sein. In verschiedenen Ausführungsbeispielen kann anhand des ermittelten Haarzustands eine Empfehlung bereitgestellt werden, z.B. bezüglich eines kosmetischen Haarbehandlungsprodukts und/oder einer Haarbehandlungsempfehlung.

Unter einem optischen Sensor ist hierin ein Sensor zu verstehen, welcher mittels optischer Elemente Licht im weiteren Sinne (UV-Licht, sichtbares Licht (auch abkürzend als VIS bezeichnet), Nahinfrarotlicht (NIR) und/oder Infrarotlicht (IR)) leitet und mittels eines Detektors (z.B. eines elektronischen Detektors, z.B. für sichtbares Licht, für NIR- oder/und IR-Licht, eines Photometers oder Ähnlichem) erfasst.

Zum direkten oder indirekten Ermitteln des Haarzustands und ggf. zum direkten oder indirekten Ermitteln der Empfehlung (z.B. des Haarbehandlungsprodukts und/oder der Haarbehandlungsempfehlung) kann die Vorrichtung oder das System in verschiedenen Ausführungsbeispielen eine Datenverarbeitungsvorrichtung aufweisen.

Der Haarzustand kann eine Haarschädigung und/oder einen Haarstatus aufweisen.

Die Haarschädigung kann in verschiedenen Ausführungsbeispielen eine oxidative Haarschädigung, eine Oberflächenschädigung oder eine mechanische Haarschädigung sein.

Der Haarstatus kann insbesondere eine Haarfarbe, einen Gehalt von einem Haarinhaltsstoff, eine Haardicke, eine Lockigkeit von Haaren und/oder einen Grauanteil von Haaren aufweisen.

Der Haarinhaltsstoff kann insbesondere Wasser, Melanine, Lipide, Aminosäuren und Mischungen daraus umfassen. Ein bevorzugter Haarinhaltsstoff, dessen Gehalt zur Ermittlung eines Haarstatus bestimmt wird, ist Wasser.

Menschliche Haare enthalten neben den 20 kanonischen Aminosäuren Glycin, Alanin, Valin, Isoleucin, Leucin, Phenylalanin, Tyrosin, Lysin, Arginin, Histidin, Asparaginsäure, Glutaminsäure, Threonin, Serin, Glutamin, Asparagin, Methionin, Cystein, Prolin und Trypthophan ferner die Aminosäuren Cystin, Ornithin und Citrullin. Entsprechend ist es bevorzugt, dass der Haarinhaltsstoff, dessen Gehalt bestimmt wird, eine Aminosäure ausgewählt aus der Gruppe bestehend aus Glycin, Alanin, Valin, Isoleucin, Leucin, Phenylalanin, Tyrosin, Lysin, Arginin, Histidin, Asparaginsäure, Glutaminsäure, Threonin, Serin, Glutamin, Asparagin, Methionin, Cystein, Prolin, Trypthophan, Cystin, Ornithin, Citrullin und Mischungen davon ist.

In verschiedenen Ausführungsbeispielen kann mithilfe eines Kamms oder einer Bürste, welche zumindest mit einem optischen Sensor versehen ist, von einem Nutzer oder einem Friseur mindestens ein Haarparameter ermittelt werden. Das heißt, dass während eines bei einer Haarpflege ohnehin stattfindenden Kämm-/Frisiervorgangs mindestens Haarparameter ermittelt werden können.

In verschiedenen Ausführungsbeispielen kann der optische Sensor beispielsweise ein Nahinfrarotsensor (NIR-Sensor) sein, z.B. ein NIR-Spektrometer oder eine NIR-Kamera, welcher eingerichtet sein kann, eine Haarschädigung und/oder einen Haarzustand zu ermitteln.

In verschiedenen Ausführungsbeispielen kann der optische Sensor beispielsweise ein Sensor für sichtbares Licht sein, z.B. ein Spektrometer oder eine Kamera für einen Spektralbereich des sichtbaren Lichts, insbesondere in einem Bereich, in welchem Fluoreszenzlicht von den Haaren emittiert wird. Dabei kann der optische Sensor eingerichtet sein, eine Haarschädigung und/oder einen Haarzustand zu ermitteln.

Der optische Sensor für das sichtbare Licht oder ein weiterer Sensor für sichtbares Licht, z.B. ein Spektrometer oder eine Farbkamera, kann in verschiedenen Ausführungsbeispielen eingerichtet sein, eine Ausgangshaarfarbe des Nutzers zu ermitteln. In einem Fall, dass die Kamera (oder eine zusätzliche Kamera) bereitgestellt ist, kann diese ferner genutzt werden, um einen weiteren Haarstatus, wie eine Haardichte und/oder eine Haardicke, zu ermitteln.

In verschiedenen Ausführungsbeispielen kann ein weiterer Sensor bereitgestellt sein, z.B. ein (Kontakt-)Mikrofon zum Ermitteln einer Oberflächenrauhigkeit des Haars, was ein Maß für eine Oberflächenschädigung des Haars darstellt.

Der optische Sensor zum Ermitteln der Haarschädigung oder des Haarstatus (zum Beispiel ein NIR-Sensor) kann in einer Basisausführung der Haarzustands-Ermittlungsvorrichtung bereitgestellt sein, wohingegen eine Premiumausführung mindestens einen weiteren Sensor aufweisen kann, beispielsweise das (Kontakt-)Mikrofon, welches in/an Zähnen des Kamms oder der Bürste angeordnet sein kann oder einen weiteren optischen Sensor. Der optische Sensor und der weitere optische Sensor können jeweils an unterschiedlichen Positionen an oder in dem Vorrichtungskörper angeordnet sein.

In verschiedenen Ausführungsbeispielen kann eine Kombination von mehreren der oben genannten Sensoren es ermöglichen, ein umfassenderes Bild des Haarzustands zu erhalten. Ein Erfassen von zwei oder mehr Haarzuständen kann als zwei- oder mehrdimensionale Messung verstanden werden, mittels welcher ein Analyseergebnis bezüglich des Haarzustands verbessert werden kann.

In verschiedenen Ausführungsbeispielen kann eine Eingabevorrichtung genutzt werden, um ein Wunschergebnis einzugeben, beispielsweise eine Wunschhaarfarbe, einen Wunsch-Pflegezustand, ein gewünschtes Styling (z.B. Locken mit einer abgestuften Lockigkeit oder ähnliches). Darüber hinaus kann die Eingabevorrichtung genutzt werden, um der Haarzustands-Ermittlungsvorrichtung weitere Informationen bereitzustellen, welche ggf. beim Ermitteln der Empfehlung berücksichtigt werden können, z.B. ob ein Produkt ggf. wasserfest sein soll, UV-Schutz enthalten soll, Alter und/oder Geschlecht des Nutzers (z.B. für einen Fall, dass ein Pflegeprodukt mit einem Duft versehen ist), usw.

In verschiedenen Ausführungsbeispielen kann die Haarzustands-Ermittlungsvorrichtung beim Ermitteln der Empfehlung, z.B. des Haarbehandlungsmittels, das Wunschergebnis berücksichtigen, derart, dass das empfohlene Produkt und/oder die empfohlene Haarbehandlung geeignet ist, am Haar des Nutzers das Wunschergebnis herbeizuführen.

In einem Fall, dass die Haarzustands-Ermittlungsvorrichtung das Mikrofon aufweist, kann das Mikrofon in verschiedenen Ausführungsbeispielen auch als Eingabemikrofon genutzt werden.

In verschiedenen Ausführungsbeispielen kann als Eingabevorrichtung an der Haarzustands-Ermittlungsvorrichtung alternativ oder zusätzlich eine andere herkömmliche Eingabevorrichtung bereitgestellt sein, z.B. Tasten, ein berührungsempfindlicher Bildschirm, etc.

Die Haarzustands-Ermittlungsvorrichtung kann in verschiedenen Ausführungsbeispielen eine Ausgabevorrichtung zum Ausgeben der Haarzustandsinformation und/oder der Empfehlung aufweisen, beispielsweise einen Lautsprecher und/oder ein Display.

In verschiedenen Ausführungsbeispielen kann die Haarzustands-Ermittlungsvorrichtung Teil eines Haarzustands-Ermittlungssystems sein, welches ferner zumindest eine Anzeigevorrichtung aufweisen kann, welche als Ausgabevorrichtung für die Haarzustandsinformation und/oder die Empfehlung genutzt werden kann.

In verschiedenen Ausführungsbeispielen, z.B. wenn die Anzeigevorrichtung ein Smartphone, Tablet, ein Smart Mirror o.ä. ist, kann die Anzeigevorrichtung ferner als Eingabevorrichtung und/oder als externe Datenverarbeitungsvorrichtung genutzt werden.

In verschiedenen Ausführungsbeispielen kann das Stylingmittel ein Haarwachs oder -gel, einen Straightener, einen Haarspray, einen Haarschaum oder ähnliches aufweisen.

In verschiedenen Ausführungsbeispielen können bei dem Ermitteln der Empfehlung Daten und/oder Erfahrungswerte von (weiteren) Nutzern, welche einen ähnlichen Haarzustand (z.B. einen ähnlichen Schädigungsgrad und/oder einen ähnlichen Haarstatus) und ggf. ein ähnliches Profil (Alter, Geschlecht, Lebensgewohnheiten, Haartyp, Ethnizität, etc.) aufweisen können, berücksichtigt werden. Ein breiter Daten- und/oder Erfahrungssatz kann dabei zu Hilfe genommen werden, um das Ergebnis zu optimieren. Das System kann ggf. als ein lernendes System gestaltet sein.

In verschiedenen Ausführungsbeispielen kann die Haarzustands-Ermittlungsvorrichtung eingerichtet sein, einen gegenwärtigen Haarschädigungszustand eines Nutzers, welcher beispielsweise mittels des NIR-Sensors ermittelt worden sein kann, von der Haarzustands-Ermittlungsvorrichtung (z.B. einem so genannten "Smarten Kamm") einer Haarbehandlungsmittel-Mischvorrichtung zu übermitteln, welche eine individuelle Haarbehandlungsmittel-Zusammensetzung ermitteln und dem Nutzer ein entsprechendes Haarbehandlungsmittel bereitstellen kann.

In verschiedenen Ausführungsbeispielen kann mittels der Haarzustands-Ermittlungsvorrichtung eine standardisierte und objektive Beurteilung des Behandlungsergebnisses ermöglicht sein. Zu dem Zweck kann mittels des in der Bürste oder dem Kamm enthaltenen optischen Sensors und ggf. weiterer Sensoren der Haarzustand des Nutzers nach einem Befolgen der Empfehlung, z.B. nach einem Anwenden des empfohlenen Produkts und/oder nach einem Durchführen der empfohlenen Behandlung, ermittelt werden.

Dadurch ermöglichte kontinuierliche, ggf. häufige, Messungen verlässlicher Haarschädigungswerte können es dem Nutzer ermöglichen, den Gesundheitszustand seiner Haare in einem zeitlichen Verlauf zu verfolgen und in einem Glauben an einen Erfolg der Behandlung bestärkt zu werden. Eine kamm- oder bürstenförmige Gestaltung des Vorrichtungskörpers, welcher dafür vorgesehen ist, das Haar zwischen Kammzinken oder Bürstenborsten entlang zu bewegen, was typischerweise derart erfolgt, dass das Haar einen Trägerbereich, der die Zinken oder Borsten trägt, berührt, ermöglicht es, den optischen Sensor im Trägerbereich anzuordnen, was bedeuten kann, dass das Haar während der Messung ohne ein Befolgen aufwändiger Instruktionen und/oder ein Bereitstellen eines Abstandshalters zuverlässig in einem festen, vorbestimmten Abstand zum Sensor angeordnet ist, was eine Einheitlichkeit und Zuverlässigkeit der Messergebnisse verbessert.

Gemäß der vorliegenden Erfindung ist die Haarzustands-Ermittlungsvorrichtung so eingerichtet, dass mittels des optischen Sensors ein Ermitteln eines Grads der (oxidativen) Haarschädigung durch eine Bestimmung eines Gehaltes an Cysteinsäure exakt ermittelbar ist. Der optische Sensor ist erfindungsgemäß eingerichtet, eine oder mehrere Aufnahmen in einem Fluoreszenzbereich und/oder in einem Nahinfrarotbereich (NIR-Bereich) zu machen.

Der Fluoreszenzbereich kann in verschiedenen Ausführungsbeispielen ein Wellenlängenbereich sein, in welchem geschädigtes Haar Eigenfluoreszenz emittiert und/oder ein Wellenlängenbereich, in welchem Fluoreszenzfarbstoffe, welche von geschädigtem Haar stärker adsorbiert werden als von ungeschädigtem Haar, Fluoreszenzlicht emittieren.

Der Nahinfrarotbereich kann in verschiedenen Ausführungsbeispielen ein Wellenlängenbereich sein, in welchem (geschädigtes) Haar Absorptionsstrukturen aufweist, z.B. in welchem Wasser oder Cysteinsäure Licht absorbiert. Bei der Nahinfrarotspektroskopie findet die Detektion im nahen Infrarot (780-2500 nm oder ca. 12.800-4.000 cm⁻¹) statt. Im Folgenden wird für Licht mit einer Wellenzahl in einem Bereich von 12.800 bis 4000 cm⁻¹ der Begriff Nahinfrarot (NIR) und für Licht mit einer Wellenzahl in einem Bereich von 3999 bis 400 cm⁻¹ der Begriff Infrarot (IR) verwendet.

Ungeschädigtes Haar kann typischerweise einen Cysteinsäuregehalt in einem Bereich von etwa 0.5% bis etwa 1% (nach Gewicht) aufweisen. Bei Vorliegen einer Schädigung, beispielsweise infolge mehrfachen Ultrablondierens und/oder anderer Schädigungsmechanismen, kann der Cysteinsäuregehalt auf über 15% (Gew.) ansteigen.

In verschiedenen Ausführungsbeispielen wird diese Eigenschaft genutzt, um den Schädigungsgrad des Haars als einen Gehalt an Cysteinsäure zu quantifizieren.

In verschiedenen Ausführungsbeispielen kann geschädigtes Haar eine Eigenfluoreszenz zeigen, welche genutzt wird, um mittels Erfassens einer Fluoreszenzintensität des Haars den Schädigungsgrad zu ermitteln.

In verschiedenen Ausführungsbeispielen kann das Haar mit einer Fluoreszenzfarbstofflösung benetzt werden, welche von geschädigtem Haar besser adsorbiert wird als von ungeschädigtem Haar, wobei die Fluoreszenzfarbstofflösung Rhodamin B, Cumarin und/oder Fluoreszein aufweisen kann.

In verschiedenen Ausführungsbeispielen kann zum Ermitteln der Fluoreszenzintensität des Haars das Haar mittels der Lichtquelle mit UV-Licht (z.B. mit Licht in einem Wellenlängenbereich von etwa 315 nm bis etwa 380 nm) belichtet werden. Zu dem Zweck kann die Sensorvorrichtung mit einer UV-Lichtquelle versehen sein. Die UV-Lichtquelle kann eine UV-LED oder eine andere geeignete Lichtquelle sein, welche klein genug ist, um in einem Körper eines Kamms oder einer Bürste untergebracht zu werden.

Während des Belichtens kann Fluoreszenzlicht registriert werden, welches von dem Haar emittiert wird. Die Fluoreszenzintensität kann anhand des registrierten Lichts ermittelt werden. Unter Einbeziehung der Fluoreszenzintensität des Haars kann dann der Schädigungsgrad des Haars ermittelt werden.

Dementsprechend kann der optische Sensor zumindest im Fluoreszenz-Wellenlängenbereich empfänglich sein. Der optische Sensor kann beispielsweise eine Kamera, ein Photometer, ein Colorimeter und/oder ein Spektrometer aufweisen oder sein. In verschiedenen Ausführungsbeispielen kann zwischen dem Haar und dem optischen Sensor in verschiedenen Ausführungsbeispielen ein Filter angeordnet sein.

Dank des technologischen Fortschritts der letzten Jahre können mittlerweile optische Sensoren (für den sichtbaren Wellenlängenbereich oder für den NIR- oder IR-Bereich) bereitgestellt werden, welche klein genug sind, um in einem Körper einer Bürste o.ä. untergebracht zu werden.

In verschiedenen Ausführungsbeispielen, beispielsweise in einem Fall, dass für den optischen Sensor, z.B. einen Detektor des optischen Sensors und/oder optische Bauelemente wie ein dispergierendes Element eines Spektrometers o.ä., mehr Platz benötigt, als nahe dem Haar (z.B. in einem Zinkenbereich eines Kamms) vorhanden ist, mindestens eine Lichtleitvorrichtung bereitgestellt sein. Dabei kann das Platz benötigende Bauelement (z.B. die Lichtquelle und/oder der Detektor) an einer Stelle der Haarzustands-Ermittlungsvorrichtung angeordnet sein, welche mehr Platz bereithält, z.B. in einem Kamm- oder Bürstengriff, und die mindestens eine Lichtleitvorrichtung kann so gestaltet sein, dass sie ein Leiten von Licht (z.B. des Lichts zum Beleuchten des Haars oder das Licht, welches mit dem Haar gewechselwirkt hat) zwischen dem Bauelement und einer Ein- oder Austrittsstelle des Lichts am Vorrichtungskörper leitet. Als Lichtleitvorrichtung können dabei herkömmliche für diesen Zweck bekannte Strukturen verwendet werden, z.B. Lichtleitfasern, Lichtleitkanäle, Spiegel und/oder sonstige optische Elemente, usw., wobei darauf zu achten ist, dass ein ggf. verwendetes lichtdurchlässiges Material für den jeweils zu leitenden Wellenlängenbereich lichtdurchlässig ist, z.B. im Fall des NIR-Lichts für den NIR-Wellenlängenbereich bis etwa 2,5 µm.

In verschiedenen Ausführungsbeispielen kann das Nahinfrarot- (NIR-) und/oder ein Infrarot- (IR- )Spektrum gewonnen werden, beispielsweise mittels ATR-(Nah-)Infrarotspektroskopie (von Englisch "attenuated total reflection", auf Deutsch "abgeschwächte Totalreflexion"). Durch eine Anwendung von mathematischen Modellen kann mittels Vermessung von Kalibrier-Haarproben, welche einen anhand eines bekannten analytischen Verfahrens ermittelten Cysteinsäuregehalt aufweisen, ein mathematisches Modell erstellt werden.

Bei einer Analyse eines am Haar des Verbrauchers aufgenommenen NIR- oder IR-Spektrums, oder zumindest eines Teils davon, kann in verschiedenen Ausführungsbeispielen das Modell eine Berechnung des Gehalts an Cysteinsäure, und damit der Haarschädigung, erlauben. Eine Analyse von zumindest einem Teil des Spektrums und eine Anwendung des Modells kann dabei mittels der Datenverarbeitungsvorrichtung, beispielsweise (mit geeigneten Apps) mittels bekannter Smartphones, Tablets, Smart Mirrors, o.ä., ausgeführt werden.

Die Lichtquelle kann in verschiedenen Ausführungsbeispielen eine NIR-Lichtquelle oder/und eine IR-Lichtquelle zum Belichten des Haars mit NIR- oder IR-Licht sein.

Das Ermitteln des Schädigungsgrads von Haar kann gemäß verschiedenen Ausführungsbeispielen entweder unter Nutzung des Nahinfrarot-Bereichs ausgeführt werden, d.h. mittels Bestrahlens des Haars mit dem Nahinfrarotlicht und Spektralanalyse von zumindest einem Teil des NIR-Lichts, nachdem dieses mit dem Haar gewechselwirkt hat, oder unter Nutzung des Infrarot-Bereichs, d.h. mittels Bestrahlens des Haars mit Infrarotlicht und Spektralanalyse von zumindest einem Teil des IR-Lichts, nachdem dieses mit dem Haar gewechselwirkt hat, oder unter Verwendung von sowohl dem Nahinfrarot- als auch dem Infrarotbereich, d.h. mittels Bestrahlens des Haars mit Nahinfrarot- und Infrarotlicht und Spektralanalyse von zumindest einem Teil des NIR- und zumindest einem Teil des IR-Lichts, nachdem dieses mit dem Haar gewechselwirkt hat.

In verschiedenen Ausführungsbeispielen kann ein vermessener Nahinfrarot (NIR)-Bereich Wellenzahlen von etwa 12500 cm⁻¹ bis etwa 4000 cm⁻¹, z.B. von etwa 5022 cm⁻¹ bis etwa 4020 cm⁻¹, aufweisen. Dieser Wellenlängenbereich kann u.a. charakteristische Oberton- und Kombinationsschwingungen von z.B. CH-, OH- und NH-Gruppen aufweisen.

In verschiedenen Ausführungsbeispielen kann der zumindest eine Teil des Nahinfrarot- und/oder Infrarotlichts einen (Infrarot-)Wellenzahlbereich von etwa 1100 cm⁻¹ bis etwa 1000 cm⁻¹, z.B. um etwa 1040 cm⁻¹, aufweisen. Hier können sich u.a. die relevanten Absorptionsbanden der zu analysierenden Komponente Cysteinsäure befinden.

In verschiedenen Ausführungsbeispielen kann anhand von Ergebnissen einer quantitativen rechnergestützten Auswertung (auch als chemometrischen Analyse bezeichnet) für eine Mehrzahl von Kalibrationshaarproben in Kombination mit mittels eines unabhängigen Verfahrens, z.B. mittels Hochdruckflüssigchromatographie, für dieselben Kalibrationshaarproben gewonnenen Werten für einen Cysteinsäuregehalt der jeweiligen Kalibrationshaarprobe, ein Kalibrationsmodell erstellt werden.

Liegt das Kalibrationsmodell vor, kann in verschiedenen Ausführungsbeispielen sehr einfach für das zu messende Haar anhand des für das aufgenommene (N)IR-Spektrum die Konzentration der Cysteinsäure (als Maß für die Haarschädigung) aus den Spektren im Vergleich mit den Kalibrationsspektren berechnet werden.

In verschiedenen Ausführungsbeispielen können für die Quantifizierung des Cysteinsäuregehalts (z.B. mittels Fluoreszenzanalyse und/oder mittels (N)IR-Spektroskopie) oder des Schädigungsgrads (z.B. mittels Interferenzreflexionsmikroskopie und/oder mittels akustischer Analyse) geeignete mathematische Modelle der Prädiktiven Analytik genutzt werden.

In verschiedenen Ausführungsbeispielen wird ein in einer Nutzung einfaches Verfahren zum bereitgestellt, welches mit Hilfe von Fluoreszenz-Detektion und/oder durch Detektion von Absorption und Methoden aus der Prädiktiven Analytik eine präzise Ermittlung eines Grads einer oxidativen Schädigung von Haar ermöglicht.

In verschiedenen Ausführungsbeispielen kann für ein Bereitstellen des ermittelten Haarzustands oder der Empfehlung eine tragbaren Datenverarbeitungsvorrichtung, auch als mobile Datenverarbeitungsvorrichtung bezeichnet, verwendet werden. Als tragbare Datenverarbeitungsvorrichtung kann dabei beispielsweise ein Smartphone, ein iPad, ein Tablet oder Laptop genutzt werden.

In verschiedenen Ausführungsbeispielen kann ein Verfahren bereitgestellt werden, welches es ermöglicht, mittels einfacher bildanalytischer Verfahren, welche sich beispielsweise unter Verwendung einer einfachen Vorrichtung (z.B. einer UV-LED, einer Weißlicht-, NIR- und/oder IR-Leuchtvorrichtung, eines Filters, eines tragbaren NIR-Sensors, eines im oder am Vorrichtungskörper der Ermittlungsvorrichtung angeordneten (NIR- und/oder VIS-)Spektrometers, ggf. in Verbindung mit einer mobilen Datenverarbeitungsvorrichtung (z.B. eines Smartphones) und ggf. eines Prädiktive-Analytik-Verfahrens eine Information bezüglich eines Haarzustands eines Nutzers und ggf. eine darauf basierende Empfehlung bereitzustellen.

In verschiedenen Ausführungsbeispielen kann eine Intensität einer Absorption des (N)IR-Lichts durch die Cysteinsäure oder eine Intensität von Fluoreszenzlicht mit Hilfe von bildanalytischen Verfahren unter normierten Bedingungen einfach erfasst werden. Durch eine Anwendung von mathematischen Modellen aus dem Bereich Prädiktive Analytik kann mittels Vermessung von Standard-Haarproben, welche einen anhand bekannter aufwändiger Verfahren ermittelten Cysteinsäuregehalt aufweisen, ein mathematisches Modell erstellt werden, welches dann bei den Haaren des Verbrauchers anhand der registrierten (N)IR-Absorption oder des registrierten Fluoreszenzlichts und der daraus ermittelten Fluoreszenzintensität eine Berechnung eines Gehalts an Cysteinsäure, und damit der Haarschädigung, erlaubt. Die Bildanalyse kann dabei beispielsweise (mit geeigneten Apps) mittels bekannter Smartphones, Tablets o.ä. ausgeführt werden.

In verschiedenen Ausführungsbeispielen erlaubt die Verwendung der mathematischen Modelle aus dem Bereich Prädiktive Analytik (wie z.B. Tree Ensembles, neuronale Netze oder Support Vector Machines) eine wesentlich exaktere Berechnung der Schädigung (welche in den Modellen eine abhängige Variable bildet), als dies mit einfachen Modellen, z.B. einer einfachen linearen Regression, möglich wäre. Die Verfahren können dabei eine Vielzahl von Input-Variablen parallel nutzen und auch nicht-lineare Zusammenhänge abbilden. In verschiedenen Ausführungsbeispielen ermöglichen diese Modelle beispielsweise eine Einbeziehung kategorialer, nicht-metrischer Input-Variablen, wie z.B. einer Haarfarbe (z.B. blond, braun, schwarz, usw.) und/oder ethnischer Zugehörigkeit eines Haartyps (z.B. kaukasisch, asiatisch, afro-amerikanisch), welche einen Einfluss auf eine resultierende NIR-Absorption oder Fluoreszenzintensität haben können. Die Input-Variablen können in verschiedenen Ausführungsbeispielen mittels der Haarzustands-Ermittlungsvorrichtung erfasst werden, z.B. die Haarfarbe unter Verwendung des optischen Sensors, sofern der optische Sensor einen in einem sichtbaren Spektralbereich empfänglichen Sensor aufweist, oder ggf. eines weiteren im Vorrichtungskörper angeordneten Sensors, sofern der weitere Sensor im sichtbaren Spektralbereich empfänglich ist.

Gemäß verschiedenen Ausführungsformen kann die Mehrzahl von NIR-absorptionsbeeinflussenden oder fluoreszenzintensitätbeeinflussenden Parametern eine Haarfarbe und/oder eine ethnische Zugehörigkeit eines Typs des Haars aufweisen. Sofern keine Sensoren zum Erfassen der NIR-absorptionsbeeinflussenden oder fluoreszenzintensitätbeeinflussenden Parameter in der Haarzustands-Ermittlungsvorrichtung vorhanden sind, können diese Parameter auch mittels der Eingabevorrichtung in der Haarzustands-Ermittlungsvorrichtung bereitgestellt werden.

Gemäß verschiedenen Ausführungsformen kann die prädiktive Analytik mindestens ein Verfahren nutzen aus einer Gruppe von Verfahren, wobei die Gruppe von Verfahren aufweist:
lineare oder multi-lineare Regression, polynome Regression, neuronale-Netze-Verfahren, Support Vector Machine-Verfahren, Entscheidungsbäume-Verfahren ("Decision Trees", "Random Forest", "Tree Ensembles") und weitere Verfahren.

In verschiedenen Ausführungsbeispielen kann die Haarzustands-Ermittlungsvorrichtung als einen weiteren Sensor einen Sensor zum Ermitteln der äußeren Haarschädigung aufweisen, z.B. einen Sensor zum Erfassen akustischer Emissionen, z.B. ein Mikrofon, z.B. ein Kontaktmikrofon.

Hierin kann der Sensor zum Erfassen akustischer Emissionen der Einfachheit halber auch als Mikrofon bezeichnet werden. Sofern nicht anders angegeben und für die beschriebene Funktion geeignet, kann der Sensor zum Erfassen akustischer Emissionen allerdings auch beispielsweise ein Beschleunigungssensor (der geeignet sein kann, Beschleunigungen infolge akustischer Emissionen in einem gewissen Frequenzbereich zu erfassen) oder ähnliches sein.

Die Haarzustands-Ermittlungsvorrichtung kann in verschiedenen Ausführungsbeispielen ein oder mehrere von außen am Vorrichtungskörper angebrachte und/oder in diesen integrierte Messsysteme für akustische Emissionen (d.h. Schallemissionen; als ein beispielhaftes Messsystem für akustische Emissionen kann ein Kontaktmikrofon der Fa. Korg genutzt werden) und/oder Messsonden für akustische Emissionen aufweisen.

In verschiedenen Ausführungsbeispielen können mittels der Sensorvorrichtung, die das Mikrofon aufweist, Signale von erzeugtem Schall oder Vibration aufgenommen werden, die bei einem Durchkämmen der Haare des Nutzers entstehen.

Die Haarzustands-Ermittlungsvorrichtung kann in verschiedenen Ausführungsbeispielen ferner einen internen oder externen Verstärker aufweisen zum Verstärken von mittels des Messsystems für akustische Emissionen gemessenen Signalen.

In verschiedenen Ausführungsbeispielen kann eine Analyse einer Haarschädigung bereitgestellt werden mittels Digitalisierens und Auswertens einer Information von dem Mikrofon mittels der elektronischen Schaltkreisvorrichtung.

Die Information kann nach einem Verarbeiten bereitgestellt werden oder sein für ein Vergleichen mit bereits aufgezeichneten Beispielen (Referenzdaten). Für die Referenzdaten kann ein Haarschädigungsgrad bekannt sein, so dass als ein Ergebnis des Vergleichs beispielsweise der Haarschädigungsgrad der ähnlichsten Referenzdaten als Ergebnis in digitaler Form bereitgestellt, z.B. zurückübermittelt, werden kann.

Das Bereitstellen der Referenzdaten und/oder das Vergleichen mit den Referenzdaten kann in verschiedenen Ausführungsbeispielen in oder mittels einer Cloud-Serverarchitektur (kurz: Cloud) erfolgen.

Mittels Verwendens verschiedener weiterer Sensoren wie z.B. Linsen, Gyroskope und Beschleunigungsmesser, kann es möglich sein, eine Position der Haarzustands-Ermittlungsvorrichtung zu ermitteln. Beispielsweise können Gyroskope und/oder Beschleunigungsmesser genutzt werden, um einen Beginn und/oder ein Ende eines Kämm- /Bürstvorgangs zu ermitteln. Unter der Annahme, dass ein Kämm-/Bürstvorgang typischerweise am Haaransatz beginnt und an den Haarspitzen endet, kann, ggf. in Kombination mit einer mittels der Sensoren ermittelten Geschwindigkeit, mit welcher die Haarzustands-Ermittlungsvorrichtung bewegt wird, eine ortsaufgelöste Ermittlung der Haarzustandsinformation ermöglicht sein, z.B. Haaransatz/mittlerer Bereich/Spitzen.

Eine Datenübermittlung von der Haarzustands-Ermittlungsvorrichtung zur externen Datenverarbeitungsvorrichtung kann in verschiedenen Ausführungsbeispielen mittels Kabel oder über bekannte Datenfunkstandards (z.B. Bluetooth, WLAN, ZigBee, Thread, NFC usw.) erfolgen.

In verschiedenen Ausführungsbeispielen wird eine Haarzustands-Ermittlungsvorrichtung zum Bereitstellen einer Information bezüglich eines Haarzustands von Haaren eines Nutzers bereitgestellt. Die Haarzustands-Ermittlungsvorrichtung kann einen Vorrichtungskörper mit mindestens einem ersten Bereich und einem zweiten Bereich aufweisen, wobei der erste Bereich und der zweite Bereich so gestaltet sind, dass die Haare des Nutzers zwischen dem ersten Bereich und dem zweiten Bereich bewegbar sind, mindestens eine optionale im oder am Vorrichtungskörper angeordnete Lichtquelle zum Beleuchten der Haare des Nutzers, mindestens einen im oder am Vorrichtungskörper angeordneten optischen Sensor zum Erfassen von Licht, welches von der Lichtquelle abgestrahlt wurde und mit den Haaren gewechselwirkt hat und/oder von Umgebungslicht, welches mit den Haaren gewechselwirkt hat, und eine im oder am Vorrichtungskörper angeordnete elektronische Schaltkreisvorrichtung,
wobei die elektronische Schaltkreisvorrichtung mit dem optischen Sensor gekoppelt ist zum Empfangen des erfassten Lichts, und wobei die elektronische Schaltkreisvorrichtung eingerichtet ist, basierend auf dem empfangenen erfassten Licht, dem Nutzer eine Information bezüglich seines Haarzustands bereitzustellen.

In verschiedenen Ausführungsbeispielen kann das Haar während des Erfassens des Lichts zwischen dem ersten und dem zweiten Bereich angeordnet sein.

In verschiedenen Ausführungsbeispielen kann das Haar während des Erfassens des Lichts zwischen dem ersten und dem zweiten Bereich bewegt werden.In verschiedenen Ausführungsbeispielen kann der Vorrichtungskörper als Kamm oder als Bürste geformt sein, wobei der erste Bereich und der zweite Bereich zwei benachbarte Kammzinken oder zwei benachbarte Borsten der Bürste aufweisen können.

In verschiedenen Ausführungsbeispielen kann der Kamm oder die Bürste einen Griff aufweisen, wobei der mindestens eine optische Sensor und ein Lichteintrittsbereich zum Zuführen des Lichts zum optischen Sensor im Griff angeordnet sein können.

In verschiedenen Ausführungsbeispielen kann die Lichtquelle eine UV-Lichtquelle aufweisen, und der mindestens eine optische Sensor kann einen Detektor für sichtbares Licht zum Erfassen von Fluoreszenzlicht aufweisen.

In verschiedenen Ausführungsbeispielen kann die Lichtquelle eine NIR-Lichtquelle aufweisen, und der mindestens eine optische Sensor kann einen NIR-Detektor zum Erfassen von Nahinfrarotlicht aufweisen.

In verschiedenen Ausführungsbeispielen kann die Lichtquelle eine VIS/NIR-Lichtquelle aufweisen, und der mindestens eine optische Sensor kann einen VIS/NIR-Detektor zum Erfassen von sichtbarem Licht und Nahinfrarotlicht aufweisen.

In verschiedenen Ausführungsbeispielen kann die elektronische Schaltkreisvorrichtung eingerichtet sein, basierend auf dem ermittelten Haarzustand eine Empfehlung zu ermitteln und dem Nutzer bereitzustellen.

In verschiedenen Ausführungsbeispielen kann die Empfehlung mindestens eine aufweisen von einer Haarpflegeproduktempfehlung, einer Haarfärbeproduktempfehlung, einer Haarstylingproduktempfehlung und einer Haarbehandlungsempfehlung.

In verschiedenen Ausführungsbeispielen kann die Haarzustands-Ermittlungsvorrichtung ferner mindestens einen weiteren Sensor zum Erfassen eines weiteren Haarzustands aufweisen.

In verschiedenen Ausführungsbeispielen kann der mindestens eine weitere Sensor ein Mikrofon zum Ermitteln einer Oberflächenrauhigkeit des Haars aufweisen.

In verschiedenen Ausführungsbeispielen kann die elektronische Schaltkreisvorrichtung eine kabellose Datenaustauschvorrichtung aufweisen.

In verschiedenen Ausführungsbeispielen wird ein Haarzustands-Ermittlungssystem bereitgestellt. Das Haarzustands-Ermittlungssystem kann eine Haarzustands-Ermittlungsvorrichtung gemäß verschiedenen Ausführungsbeispielen und eine Anzeigevorrichtung aufweisen,
wobei die mindestens eine Haarzustands-Ermittlungsvorrichtung eingerichtet sein kann, der Anzeigevorrichtung die Haarzustandsinformation mittels der Datenaustauschvorrichtung zu übermitteln.

In verschiedenen Ausführungsbeispielen kann die Anzeigevorrichtung einen Computerbildschirm, ein Smartphone, ein Tablet, ein iPad, einen Smart Mirror oder einen Laptop aufweisen.

In verschiedenen Ausführungsbeispielen wird ein Verfahren zum Bereitstellen einer Haarzustandsinformation bezüglich Haaren eines Nutzers bereitgestellt. Das Verfahren kann ein Anordnen einer Haarzustands-Ermittlungsvorrichtung gemäß verschiedenen Ausführungsbeispielen aufweisen, derart, dass die Haare des Nutzers einem Lichteintrittsbereich der Haarzustands-Ermittlungsvorrichtung zugewandt sind, während eines optionalen Beleuchtens des Haars mittels der Lichtquelle, ein Erfassen zumindest eines Teils des Lichts, welches mit den Haaren gewechselwirkt hat und/oder von Umgebungslicht, welches mit den Haaren gewechselwirkt hat, gegebenenfalls während des Beleuchtens, und ein Bereitstellen einer Haarzustandsinformation bezüglich der Haare des Nutzers basierend auf dem erfassten Licht.

In verschiedenen Ausführungsbeispielen kann der Lichteintrittsbereich eingerichtet sein, das Licht dem mindestens einen optischen Sensor zuzuführen.

In verschiedenen Ausführungsbeispielen kann das Verfahren ferner ein Ermitteln mindestens einer Empfehlung basierend auf dem ermittelten Haarzustand aufweisen.

In verschiedenen Ausführungsbeispielen kann die Empfehlung mindestens eine aufweisen von einer Haarpflegeproduktempfehlung, einer Haarfärbeproduktempfehlung, einer Haarstylingproduktempfehlung und einer Haarbehandlungsempfehlung.

Gemäß einer weiteren Ausführungsform ist die Haarzustands-Ermittlungsvorrichtung ausgeführt, nach dem Ausgeben von einer Empfehlung, insbesondere einer Produktempfehlung, eine Eingabe von einem Nutzer entgegen zu nehmen und basierend auf dieser Eingabe eine Aktion betreffend das angezeigte/ausgegebene Produkt zu veranlassen.

Die Aktion kann sich beispielsweise darauf beziehen, dass dem Nutzer ein Produkt, beispielsweise ein Haarpflegeprodukt, ein Haarfärbeprodukt und/oder ein Haarstylingprodukt zum Kauf angeboten wird und der Nutzer über eine Eingabe den Kauf in die Wege leiten kann. Neben dem Kauf von Produkten können dem Nutzer auch weitergehende Informationen zum käuflichen Erwerb angeboten werden. Diese weitergehenden Informationen können sich auf detailliertere Behandlungs- und Anwendungshinweise beziehen. Die Haarzustands-Ermittlungsvorrichtung empfängt beispielsweise die Anfrage, dass der Nutzer das Produkt erwerben möchte, speichert die Anfrage und/oder übermittelt die Anfrage an ein Handelsunternehmen, welches die Produkte vertreibt. Der Nutzer wird durch die Haarzustands-Ermittlungsvorrichtung, beispielsweise über die Ausgabevorrichtung, aufgefordert, seine persönlichen Daten (Adresse, Bankinformationen, Versendungspräferenz, etc.) über die Eingabeeinheit einzugeben.

Alternativ kann dem Nutzer ausgegeben werden, wo, beispielsweise in einem Drogeriemarkt, in einem Friseursalon, in einer Apotheke, etc. in seiner Nähe, er das ausgegebene, beispielsweise angezeigte, Produkt vor Ort erwerben kann.

Immer mehr Kunden wünschen sich ein individuell auf ihre Bedürfnisse abgestimmtes Produkt. Entsprechend kann dem Nutzer ein individuell für ihn hergestelltes Produkt empfohlen und ein Bestellvorgang, beispielsweise durch Aufrufen einer Internetseite eines Herstellers von individuellen Haarbehandlungsprodukten, eingeleitet werden.

Dabei kann es sich um ein speziell für den einen Nutzer hergestelltes Haarbehandlungsprodukt oder ein sogenanntes "mass customized" Produkt handeln. Bei einem "mass customized" Produkt kann durch Variation von wenigen, aus Kundensicht jedoch entscheidenden, Merkmalen eines Produkts, eine Individualisierung erreicht werden. Bevorzugt basieren diese "mass customized" Produkte auf dem Konzept der Modularisierung, das heißt, das Produkt kann aus diversen Modulen/Bausteinen individuell zusammengestellt werden.

Zwischen den vielen unterschiedlichen Merkmalen/Inhaltsstoffen eines Produktes bestehen oftmals zahlreiche Abhängigkeiten, die als "Gebote" oder "Verbote" ausgedrückt werden können. Um eine eindeutige Produktdefinition zu erhalten, kann es vorteilhaft sein, dass der Bestellvorgang mit Hilfe eines Produktkonfigurators abläuft. Dieser Konfigurator hilft dem Nutzer bei der Auswahl der Merkmale/Inhaltsstoffe und weist ihn auf die zulässigen/unzulässigen Merkmalskombinationen hin, wobei letztere dann nicht ausgewählt werden können.

Bei Haarbehandlungsprodukten umfassen die relevanten Produktmerkmale insbesondere die chemischen Inhaltsstoffe der Mittel, die physikalischen Eigenschaften der Mittel und die Konfektionsart der Mittel. Mit Hilfe eines Produktkonfigurators kann beispielsweise die Auswahl chemisch und/oder physikalisch inkompatibler Inhaltsstoffe oder die Auswahl für den ermittelten Haarzustand ungeeigneter Inhaltsstoffe vermieden werden. Umgekehrt kann die Auswahl für den ermittelten Haarzustand geeigneter Inhaltsstoffe durch den Produktkonfigurator vorgegeben oder vorgeschlagen werden.

Ebenso ist es möglich, ein individuelles Haarbehandlungsprodukt vor Ort, das heißt zum Beispiel in einem Friseursalon oder an einem Verkaufspunkt von Haarbehandlungsprodukten, wie beispielsweise ein Drogeriemarkt, mittels einer Mischvorrichtung, vorzugsweise einer intelligenten Mischvorrichtung (Smart Mixer), herzustellen.

In verschiedenen Ausführungsbeispielen kann der ermittelte Haarzustand mindestens einen aufweisen von einer Haarschädigung und einem Haarstatus.

Die Haarschädigung kann in verschiedenen Ausführungsbeispielen eine oxidative Haarschädigung sein.

Der Haarstatus kann in verschiedenen Ausführungsbeispielen eine Haarfarbe, einen Wassergehalt von Haaren und/oder eine Haardicke aufweisen.

Gemäß einem weiteren Aspekt ist ein Verfahren zum Ermitteln eines Behandlungsmittels basierend auf dem ermittelten Haarzustand angegeben. Dieses Verfahren weist die folgenden Schritte auf: Heranziehen des ermittelten Haarzustandes und Auswählen eines Behandlungsprodukts für Haar basierend auf dem ermittelten Haarzustand und Ausgeben von Informationen über das ausgewählte Behandlungsprodukt.

Gemäß einem weiteren Aspekt ist ein Computerprogrammprodukt angegeben, welches ausgeführt ist, die Verfahren wie hierin beschrieben auszuführen, wenn sie auf einer Vorrichtung wie ebenfalls hierin beschrieben ausgeführt werden.

Ausführungsbeispiele der Erfindung sind in den Figuren dargestellt und werden im Folgenden näher erläutert.

Es zeigen
Figur 1A und 1B jeweils eine schematische Darstellung einer Haarzustands-Ermittlungsvorrichtung gemäß verschiedenen Ausführungsbeispielen;
Figur 2A, 2B, 2C und 2D jeweils eine schematische Darstellung eines Haarzustands-Ermittlungssystems gemäß verschiedenen Ausführungsbeispielen;
Figur 3A eine schematische Veranschaulichung einer Nutzung einer Haarzustands-Ermittlungsvorrichtung gemäß verschiedenen Ausführungsbeispielen;
Figur 3B eine schematische Veranschaulichung einer Nutzung eines Haarzustands-Ermittlungssystems gemäß verschiedenen Ausführungsbeispielen; und
Figur 4 ein Ablaufdiagramm, welches ein Verfahren zum Ermitteln eines Haarzustands gemäß verschiedenen Ausführungsbeispielen darstellt.

In der folgenden ausführlichen Beschreibung wird auf die beigefügten Zeichnungen Bezug genommen, die Teil der vorliegenden Anmeldung bilden und in denen zur Veranschaulichung spezifische Ausführungsformen gezeigt sind, in denen die Erfindung ausgeübt werden kann. Es versteht sich, dass andere Ausführungsformen benutzt und strukturelle oder logische Änderungen vorgenommen werden können, ohne von dem Schutzumfang der vorliegenden Erfindung abzuweichen. Es versteht sich, dass die Merkmale der hierin beschriebenen verschiedenen beispielhaften Ausführungsformen miteinander kombiniert werden können, sofern nicht spezifisch anders angegeben. Die folgende ausführliche Beschreibung ist deshalb nicht in einschränkendem Sinne aufzufassen, und der Schutzumfang der vorliegenden Erfindung wird durch die angefügten Ansprüche definiert.

In der vorliegenden Beschreibung werden die Begriffe Prädiktive Analytik, Predictive Analytics, Big Data und Data Mining synonym verwendet.

Bei einem hierin genannten Smartphone ist dies, sofern aus dem Kontext nicht etwas Anderes hervorgeht, als stellvertretend zu verstehen für alle ähnlichen Arten von tragbarer Datenverarbeitungsvorrichtung, d.h. Smartphones, Tablets, iPads, Laptops, usw. Sinngemäßes gilt für Smartphonekameras und Ähnliches.

Unter einem Kamm ist hierin eine Vorrichtung zum Kämmen von Haaren zu verstehen, bei welcher Kammzinken als im Wesentlichen eindimensionale Anordnung gebildet sind.

Unter einer Bürste ist hierin eine Vorrichtung zum Bürsten von Haaren zu verstehen, bei welcher Borsten als im Wesentlichen zweidimensionale Anordnung gebildet sind.

Unter einem Haarfärbemittel ist hierin ein Mittel zum Ändern einer Haarfarbe zu verstehen. Das Haarfärbemittel kann also entweder eine Coloration zum Erzeugen einer Haarfarbe (z.B. schwarz, braun oder rot) sein, oder ein Blondierungsmittel zum Entfernen/Aufhellen einer Haarfarbe durch Zerstörung von Melanin.

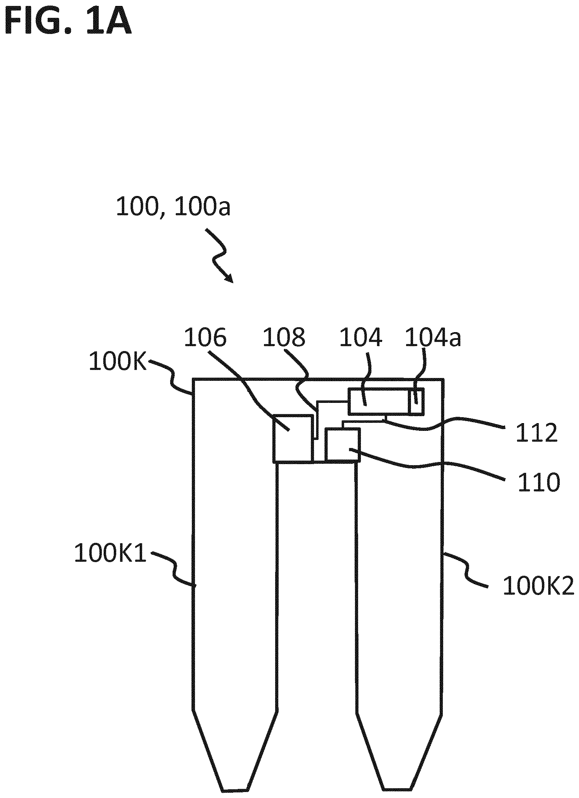

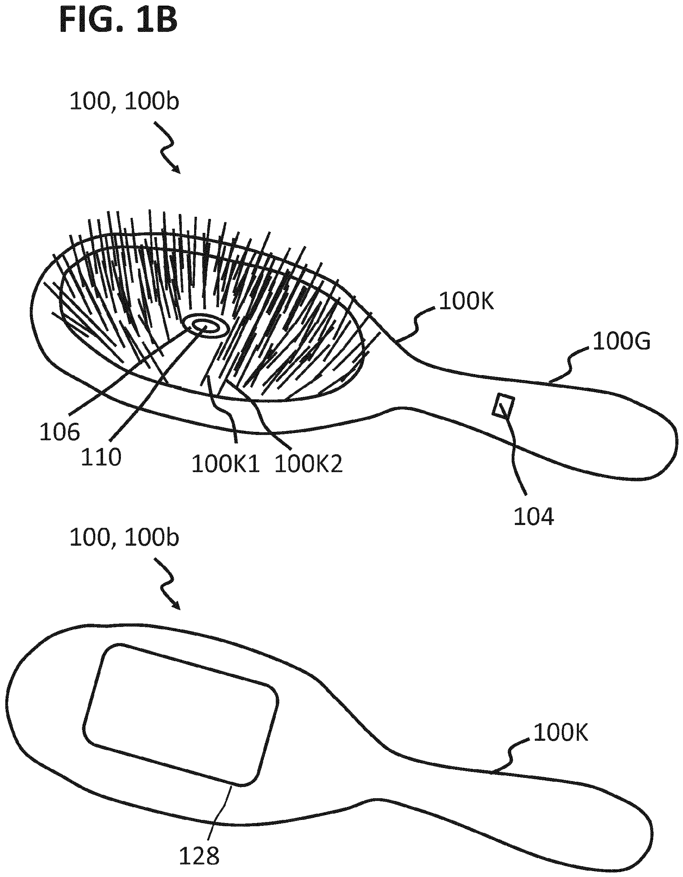

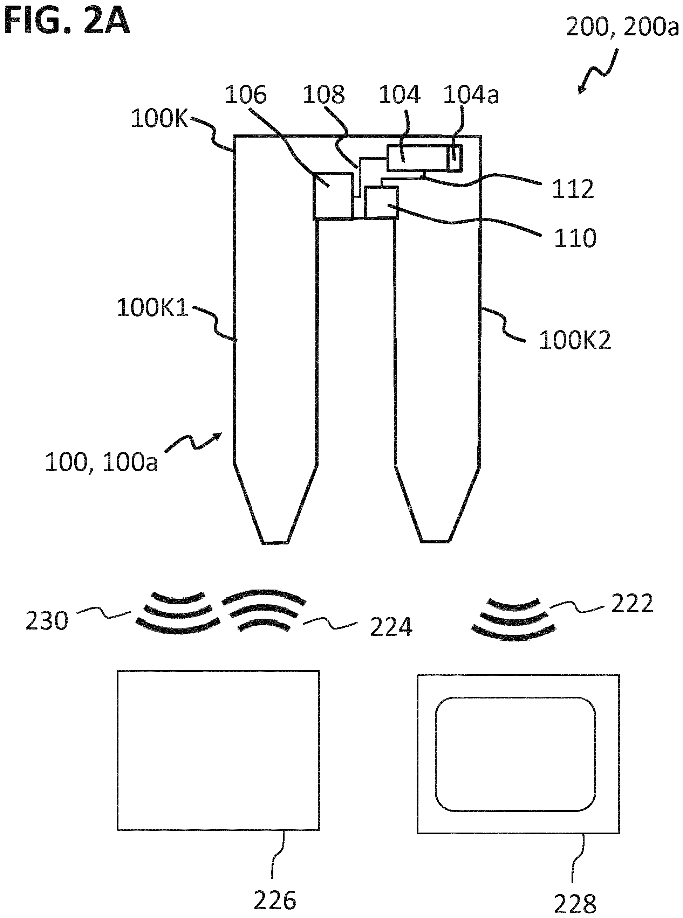

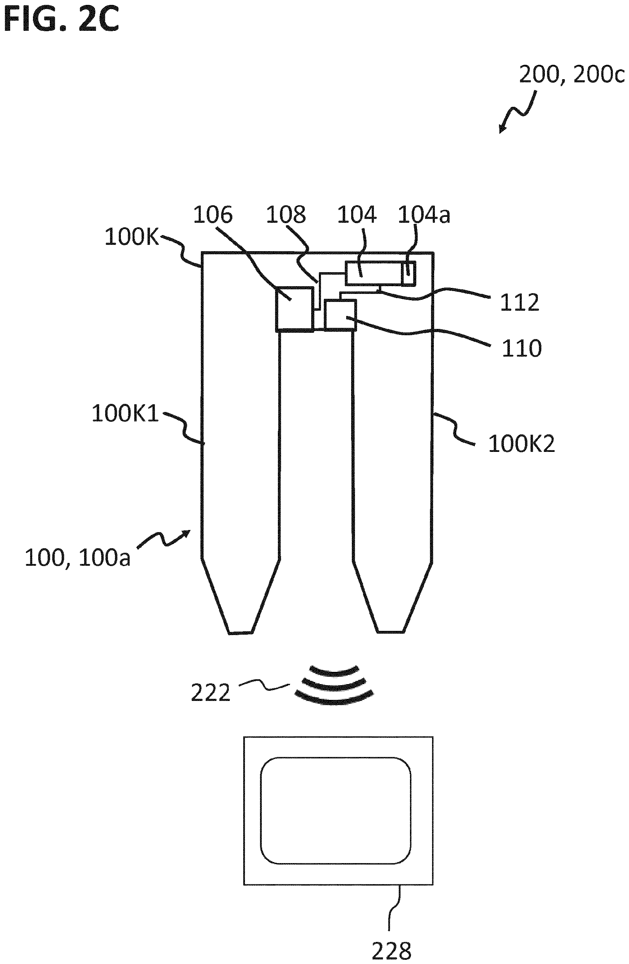

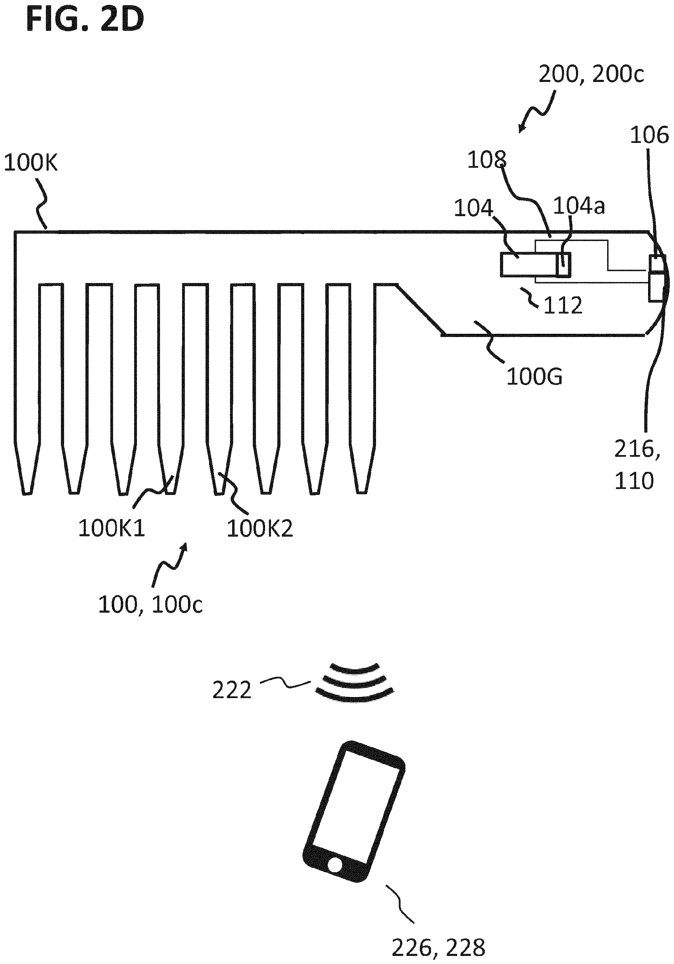

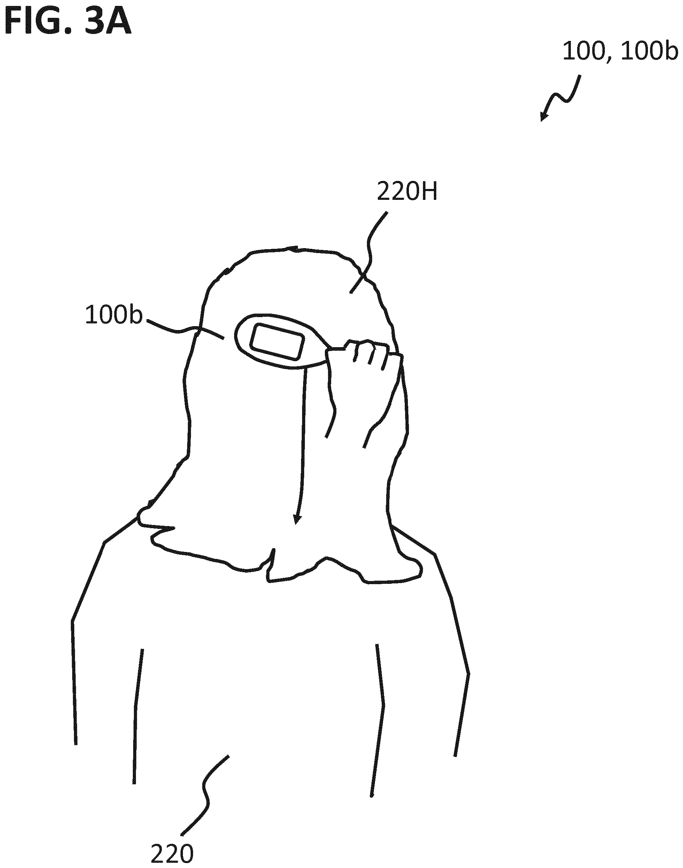

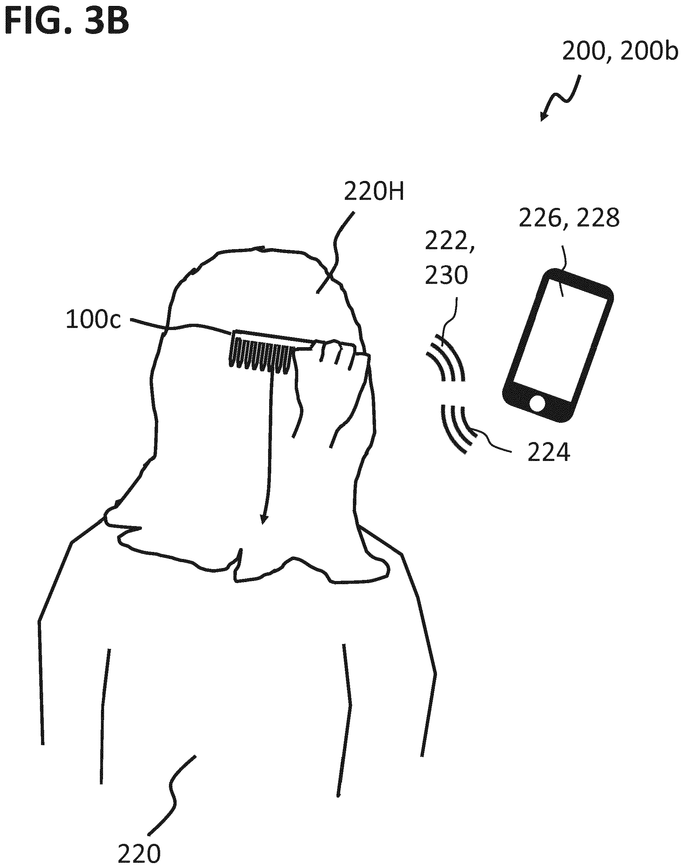

FIG. 1A und FIG. 1B zeigen jeweils eine schematische Darstellung einer Haarzustands-Ermittlungsvorrichtung 100 gemäß verschiedenen Ausführungsbeispielen, FIG. 2A, FIG. , FIG. 2C und FIG. 2D zeigen jeweils eine schematische Darstellung eines Haarzustands-Ermittlungssystems 200 gemäß verschiedenen Ausführungsbeispielen, FIG. 3A zeigt eine schematische Veranschaulichung einer Nutzung einer Haarzustands-Ermittlungsvorrichtung gemäß verschiedenen Ausführungsbeispielen und FIG. 3B zeigt eine schematische Veranschaulichung einer Nutzung eines Haarzustands-Ermittlungssystems gemäß verschiedenen Ausführungsbeispielen.

Unterschiedliche Ausführungsbeispiele der Haarzustands-Ermittlungsvorrichtung 100 und des Haarzustands-Ermittlungssystems 200 sind mit nachgestellten Buchstaben gekennzeichnet.

In verschiedenen Ausführungsbeispielen kann die Haarzustands-Ermittlungsvorrichtung 100 einen Vorrichtungskörper 100K mit mindestens einem ersten Bereich 100K1 und einem zweiten Bereich 100K2 aufweisen, wobei der erste Bereich 100K1 und der zweite Bereich 100K2 so gestaltet sein können, dass die Haare 220H des Nutzers 220 zwischen dem ersten Bereich 100K1 und dem zweiten Bereich 100K2 bewegbar sind.

Die Vorrichtung 100 kann ferner in verschiedenen Ausführungsbeispielen mindestens eine im oder am Vorrichtungskörper 100K angeordnete Lichtquelle 106 zum Beleuchten von zwischen dem ersten Bereich und dem zweiten Bereich bewegten Haaren 220H des Nutzers 220.

Die Vorrichtung 100 kann ferner in verschiedenen Ausführungsbeispielen mindestens einen im oder am Vorrichtungskörper 100K angeordneten optischen Sensor 110 zum Erfassen von Licht, welches von der Lichtquelle 106 abgestrahlt wurde und mit den zwischen dem ersten Bereich und dem zweiten Bereich bewegten Haaren 220H gewechselwirkt hat, aufweisen.

Die Vorrichtung 100 kann ferner in verschiedenen Ausführungsbeispielen eine im oder am Vorrichtungskörper 100K angeordnete elektronische Schaltkreisvorrichtung 104 aufweisen, wobei die elektronische Schaltkreisvorrichtung 104 mit dem optischen Sensor 110 gekoppelt sein kann zum Empfangen eines mittels des erfassten Lichts erzeugten Signals, und wobei die elektronische Schaltkreisvorrichtung 104 eingerichtet sein kann, basierend auf dem empfangenen Signal, eine Information bezüglich des Haarzustands des Nutzers 220 zu ermitteln und bereitzustellen.

Gemäß der vorliegenden Erfindung weist die Haarzustands-Ermittlungsvorrichtung 100 einen optischen Sensor 110 zum Ermitteln einer Haarschädigung und/oder eines Haarzustands des Haars 220H auf.

Gemäß der vorliegenden Erfindung weist die Haarzustands-Ermittlungsvorrichtung 100 einen optischen Sensor 110 zum Ermitteln eines Cysteinsäuregehalts des Haars 220H auf.

Gemäß der vorliegenden Erfindung weist die Haarzustands-Ermittlungsvorrichtung 100 einen optischen Sensor 110 zum Ermitteln eines Wassergehalts des Haars 220H auf.

In verschiedenen Ausführungsbeispielen kann die Haarzustands-Ermittlungsvorrichtung 100 einen optischen Sensor 110 zum Ermitteln einer Farbe des Haars 220H aufweisen.

Der optische Sensor 110e zum Ermitteln eines Cysteinsäuregehalts des Haars 220H kann, wie oben beschrieben, in verschiedenen Ausführungsbeispielen eine Kamera und/oder ein Spektrometer zum Erfassen von Fluoreszenzlicht in einem Wellenlängenbereich sichtbaren Lichts aufweisen, wobei das Fluoreszenzlicht von dem Haar 220H bei einem Bestrahlen mit UV-Licht abgestrahlt wird.

Zum Bestrahlen mit dem UV-Licht kann die Haarzustands-Ermittlungsvorrichtung 100 eine Lichtquelle 106 aufweisen, z.B. eine UV-Lampe, eine UV-LED oder ähnliches, welche geeignet ist, UV-Licht in einem Wellenlängenbereich abzustrahlen, welches geschädigtes Haar zum Abstrahlen von Fluoreszenzlicht anregt.

Das Fluoreszenzlicht kann eine Eigenfluoreszenz des geschädigten Haars 220H sein, und/oder eine Fluoreszenz von in dem Haar 220H adsorbiertem Fluoreszenzfarbstoff. Das Haar 220H kann vom Kopf entfernt worden sein, beispielsweise zum Benetzen des Haars mit einer Fluoreszenzfarbstofflösung und/oder für das Registrieren des Fluoreszenzlichts, oder das Haar kann am Kopf des Nutzers verbleiben, beispielsweise bei einem Registrieren der Eigenfluoreszenz des Haars.

Das Bestrahlen, Erfassen und ein Auswerten des Erfassten Sensorsignals kann in verschiedenen Ausführungsbeispielen erfolgen wie oben beschrieben. Damit kann als Haarzustandsinformation ein Haarschädigungsgrad ermittelt werden.

Der optische Sensor 110 zum Ermitteln eines Cysteinsäuregehalts oder eines anderen Inhaltsstoffgehalts des Haars 220H kann, wie oben beschrieben, in verschiedenen Ausführungsbeispielen eine Kamera und/oder ein Spektrometer zum Erfassen von (Nah- )Infrarotlicht aufweisen, wobei das (Nah-)infrarotlicht von dem Haar 220H abgestrahlt wird, während das Haar 220H mit dem (Nah-)infrarotlicht bestrahlt wird und mit diesem wechselwirkt, z.B. einen Teil davon absorbiert.

Zum Bestrahlen mit dem (N)IR-Licht kann die Lichtquelle 106 eine (N)IR-Lampe aufweisen, welche geeignet ist, (N)IR-Licht zumindest in einem Wellenlängenbereich abzustrahlen, in welchem Cysteinsäure Licht absorbiert.

Zum Steuern der Lichtquelle 106 kann in verschiedenen Ausführungsbeispielen die Lichtquelle 106 mit der elektronischen Schaltkreisvorrichtung 104 verbunden sein, beispielsweise mittels einer Verbindung 108.

Das Bestrahlen, Erfassen und ein Auswerten des erfassten Sensorsignals kann in verschiedenen Ausführungsbeispielen erfolgen wie oben beschrieben. Damit kann als Haarzustandsinformation ein Haarschädigungsgrad und/oder ein Haarstatus ermittelt werden.

Ein Austrittsbereich der Lichtquelle 106 aus dem Vorrichtungskörper und ein Eintrittsbereich des optischen Sensors 110 können in verschiedenen Ausführungsbeispielen relativ zueinander derart angeordnet sein, dass Licht, welches von der Lichtquelle 106 zum Haar 220H abgestrahlt wird, nach dem Wechselwirken mit dem Haar 220H zumindest teilweise so in Richtung zum optischen Sensor 110 abgestrahlt wird, dass es vom optischen Sensor 110 erfassbar ist.

Der als Spektrometer gebildete optische Sensor 110 kann in verschiedenen Ausführungsbeispielen eingerichtet sein, sowohl das Fluoreszenzlicht oder (N)IR-Licht auswertbar zu erfassen als auch ein Ermitteln der Haarfarbe oder beispielsweise des Wassergehalts des Haars zu ermöglichen.

In verschiedenen Ausführungsbeispielen kann der optische Sensor 110 benachbart zur Lichtquelle 106 angeordnet sein, wie beispielhaft in FIG. 1A, FIG. 2A, FIG. 2C und 2D für die Haarzustands-Ermittlungsvorrichtung 100a dargestellt.

In verschiedenen Ausführungsbeispielen kann der optische Sensor 110 innerhalb der Lichtquelle 106 (z.B. von der Lichtquelle umgeben) angeordnet sein, wie beispielhaft in FIG. 1B für die Haarzustands-Ermittlungsvorrichtung 100b dargestellt (ein Teil der Borsten ist in FIG. 1B nicht dargestellt, um den Blick auf den Sensor 110 und die Lichtquelle 106 freizugeben).

In verschiedenen Ausführungsbeispielen, beispielsweise bei geringem Platzangebot wie beispielsweise im Kämmbereich eines Kamms, können die Lichtquelle 106 und/oder der optische Sensor 110 entfernt vom Austritts- oder Eintrittsbereich angeordnet sein. Wie oben beschrieben, kann in verschiedenen Ausführungsbeispielen die Lichtquelle 106 entfernt vom Lichtaustrittsbereich angeordnet sein, beispielsweise an einer Stelle des Vorrichtungskörpers 100K, welcher mehr Platz bereithält, und das abzustrahlende Licht kann mittels einer Lichtleitvorrichtung zum Lichtaustrittsbereich geleitet werden.

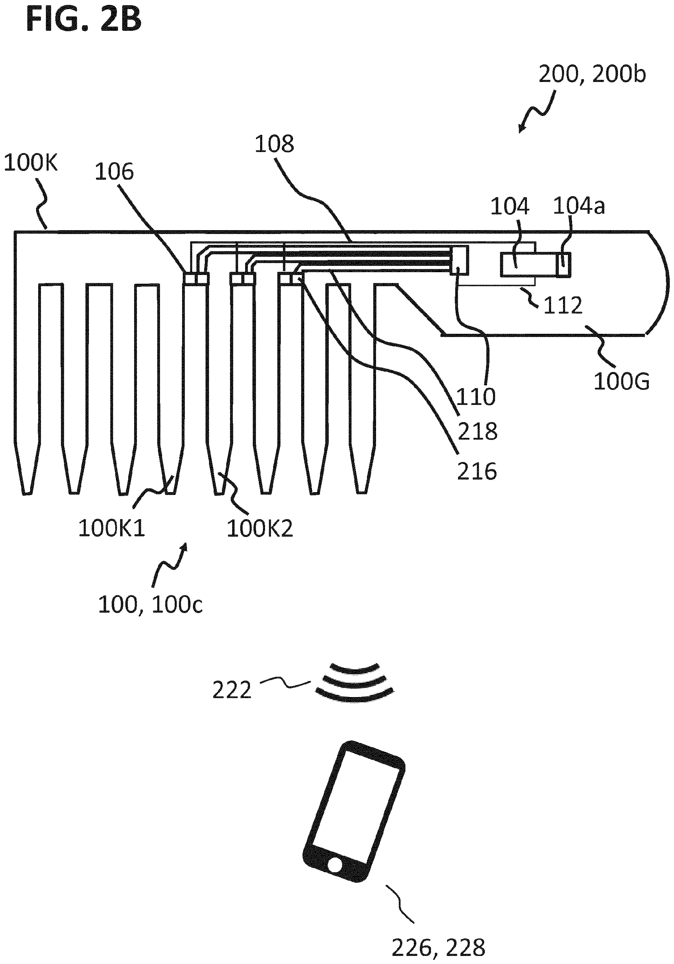

In verschiedenen Ausführungsbeispielen, wie beispielhaft in FIG. 2B für die Haarzustands-Ermittlungsvorrichtung 100c dargestellt, kann der optische Sensor 110 entfernt von einem Lichteintrittsbereich 216 angeordnet sein, beispielsweise an einer Stelle des Vorrichtungskörpers 100K, welcher mehr Platz bereithält, und das in den Lichteintrittsbereich 216 eintretende Licht kann mittels einer Lichtleitvorrichtung 218 zum optischen Sensor 110 (z.B. dem Detektor) geleitet werden.

In verschiedenen Ausführungsbeispielen, wie in FIG. 2D veranschaulicht, kann der mindestens eine optische Sensor 110 (und gegebenenfalls die Lichtquelle 106) im Griff 100G des Vorrichtungskörpers 100K angeordnet sein, und auch der Lichteintrittsbereich 216 zum Zuführen des Lichts vom Haar zum optischen Sensor 110 kann im Griff angeordnet sein, beispielsweise an einem Griffende. Zum Ermitteln des Haarzustands kann der Kamm oder die Bürste so angeordnet werden, dass der Lichteintrittsbereich 216 dem Haar 102H zugewandt ist, im Ausführungsbeispiel aus FIG. 2D also beispielsweise mit dem Griffende in Richtung zum Haar 102H. Damit kann beispielsweise ermöglicht werden, dass ein Friseur während eines Frisiervorgangs den Kamm oder die Bürste kurz umdreht und eine Messung zum Erfassen des Haarzustands vornimmt.

In verschiedenen Ausführungsbeispielen kann ein Anordnen des Lichteintrittsbereichs 216 an einer Stelle des Vorrichtungskörpers 100K, an welcher der erste Bereich 100K1 und der zweite Bereich 100K2 am Vorrichtungskörper 100K angebracht sind, d.h. zwischen den Zinken des Kamms oder zwischen den Borsten der Bürste, ermöglichen, einen im Wesentlichen konstanten Abstand zwischen dem Lichteintrittsbereich 216 und den zwischen dem ersten Bereich 100K1 und dem zweiten Bereich 100K2 angeordneten Haaren 220H zu gewährleisten, ohne den Nutzer 220 instruieren zu müssen, wie er die Haarzustands-Ermittlungsvorrichtung 100 relativ zu seinen Haaren zu positionieren hat, denn üblicherweise erfolgt ein Kämm- oder Bürstvorgang derart, dass das Haar mit dem Vorrichtungskörper 100K zwischen den Zinken 100K1, 100K2 oder Borsten in Kontakt ist.

Damit kann ohne zusätzlichen Aufwand eine Schwankungsbreite in den Messwerten verkleinert, d.h. zuverlässigere Haarzustands-Messergebnisse erzielt werden.

In verschiedenen Ausführungsbeispielen kann eine Mehrzahl von Lichtquellen 106 und/oder eine Mehrzahl von Lichtaustrittsbereichen bereitgestellt sein.

In verschiedenen Ausführungsbeispielen kann eine Mehrzahl von optischen Sensoren 110 und/oder eine Mehrzahl von Lichteintrittsbereichen 216 bereitgestellt sein, wobei die Mehrzahl von optischen Sensoren 110 eingerichtet sein kann, dieselbe Art von Signal zu erfassen, z.B. entweder Fluoreszenzlicht oder (Nah-)Infrarotlicht oder sichtbares Licht. Alternativ kann die Mehrzahl von optischen Sensoren 110 eingerichtet sein, unterschiedliche Arten von Signalen zu erfassen, z.B. Fluoreszenzlicht und (Nah-)Infrarotlicht oder sichtbares Licht und (Nah-)Infrarotlicht oder Fluoreszenzlicht, sichtbares Licht und (Nah-)Infrarotlicht.

In verschiedenen Ausführungsbeispielen kann ferner mindestens ein weiterer optischer Sensor bereitgestellt sein (nicht dargestellt), welcher eingerichtet sein kann, Licht zu erfassen, um mindestens einen weiteren Sensorwert zu erfassen, welcher beim Ermitteln des Haarzustands herangezogen werden kann. Der mindestens eine weitere optische Sensor kann beispielsweise eine Farbkamera und/oder ein Spektrometer aufweisen, welche/s eingerichtet sein kann, Licht in mehreren sichtbaren Wellenlängenbereichen zu erfassen, was ein Ermitteln einer Haarfarbe des Nutzers 220 ermöglicht, beispielsweise wie oben beschrieben.

In verschiedenen Ausführungsbeispielen kann die Haarzustands-Ermittlungsvorrichtung eine Ausgabevorrichtung 128 aufweisen, welche eingerichtet sein kann, den ermittelten Haarzustand und ggf. eine mittels des ermittelten Haarzustands ermittelte Empfehlung bereitzustellen.

Die Ausgabevorrichtung 128 kann in verschiedenen Ausführungsbeispielen eine Anzeigevorrichtung 128 (auch als Display bezeichnet) sein, wie beispielhaft in FIG. 1B dargestellt. Alternativ oder zusätzlich kann die Haarzustands-Ermittlungsvorrichtung eine andere Ausgabevorrichtung 128 aufweisen, beispielsweise einen Lautsprecher.

Mittels der Ausgabevorrichtung 128 kann der ermittelte Haarzustand (z.B. der ermittelte Haarschädigungsgrad) und ggf. eine mittels des ermittelten Haarzustands ermittelte Empfehlung, z.B. eine Empfehlung für ein Haarfärbeprodukt, ein Haarpflegeprodukt, Haarstyling und/oder eine Haarbehandlung, bereitgestellt werden.

In verschiedenen Ausführungsbeispielen kann eine externe Ausgabevorrichtung 228 bereitgestellt sein, welche mit der Haarzustands-Ermittlungsvorrichtung 100 zusammen ein Haarzustands-Ermittlungssystem 200 bilden kann, wie dies beispielhaft in FIG. 2A, 2B, 2C, 2D und FIG. 3B dargestellt ist.

Die elektronische Schaltkreisvorrichtung 104 kann, beispielsweise mittels der Vorrichtung 104a zum kabellosen Übertragen von Daten, die anzuzeigenden Daten an die Ausgabevorrichtung 228 als Signal 222 übermitteln.

Dabei kann die Ausgabevorrichtung 228, beispielsweise ein Display 228, in verschieden Ausführungsbeispielen, wie in FIG. 2A und FIG. 2C dargestellt, lediglich eine Ausgabefunktion erfüllen.

In verschiedenen Ausführungsbeispielen, wie z.B. in FIG. 2B und in FIG. 3B dargestellt, kann die Ausgabevorrichtung 228 Teil der externen Datenverarbeitungsvorrichtung 226 sein. In dem Fall kann die externe Datenverarbeitungsvorrichtung 226 zusätzlich zu einer Ausgabefunktion gegebenenfalls zusätzliche Aufgaben erfüllen, beispielsweise als die Eingabevorrichtung, zum Ermitteln des Haarzustands und/oder der Empfehlung, zum Speichern von Ergebnissen zum Erstellen einer zeitlichen Entwicklung, usw.

In verschiedenen Ausführungsbeispielen kann die Haarzustands-Ermittlungsvorrichtung eine Eingabevorrichtung aufweisen. Die Eingabevorrichtung ist nur für solche Beispiele in den Figuren dargestellt, in welchen das in die Vorrichtung 100 integrierte Display 128 oder ein externes Display 228 auch als Eingabevorrichtung genutzt werden kann, beispielsweise im Fall eines berührungsempfindlichen Displays. Die Eingabevorrichtung kann beispielsweise genutzt werden, um ein Wunschergebnis einzugeben, beispielsweise eine Wunschhaarfarbe, einen Wunsch-Pflegezustand, ein gewünschtes Styling (z.B. Locken mit einer abgestuften Lockigkeit oder ähnliches). Ferner kann die Eingabevorrichtung genutzt werden, um der Haarzustands-Ermittlungsvorrichtung weitere Informationen bereitzustellen, welche ggf. beim Ermitteln der Empfehlung berücksichtigt werden können, z.B. ob ein Produkt ggf. wasserfest sein soll, einen UV-Filter enthalten soll, Alter, Ethnizität und/oder Geschlecht des Nutzers, usw.

In verschiedenen Ausführungsbeispielen kann der mindestens eine weitere Sensor ein Mikrofon zum Ermitteln einer Oberflächenrauhigkeit des Haars, beispielsweise wie oben beschrieben, aufweisen.

Anhand der ermittelten Oberflächenrauhigkeit kann in verschiedenen Ausführungsbeispielen der (Oberflächen-)Schädigungsgrad des Haars ermittelt werden, beispielsweise wie oben beschrieben.

In verschiedenen Ausführungsbeispielen kann oder können das registrierte Licht und/oder das registrierte Geräusch oder ggf. ein anderer oder weiterer erfasster Sensorwert als Rohdaten und/oder in verarbeiteter Form, beispielsweise als Digitalfoto oder eine andere Quantifizierung des registrierten Lichts, als Audiodatei, Fouriertransformation o.ä. an die elektronische Schaltkreisvorrichtung 104 übertragen werden, beispielsweise mittels einer Verbindung 112.

In verschiedenen Ausführungsbeispielen kann die elektronische Schaltkreisvorrichtung 104 eingerichtet sein, den Haarzustand, z.B. den Haarschädigungsgrad und/oder den Haarstatus, und/oder die Empfehlung direkt, d.h. selbst, zu ermitteln, beispielsweise mittels einer Software, beispielsweise wie oben beschrieben.

In verschiedenen Ausführungsbeispielen kann die elektronische Schaltkreisvorrichtung 104 eingerichtet sein, den Haarzustand, z.B. den Haarschädigungsgrad und/oder den Haarstatus, und/oder die Empfehlung indirekt zu ermitteln, beispielsweise mittels eines Übermittelns der Sensor-Rohdaten und/oder von teilweise ausgewerteten Sensordaten und/oder von einem Haarzustand an eine externe Datenverarbeitungsvorrichtung 226, beispielsweise an eine tragbare Datenverarbeitungsvorrichtung (z.B. ein Smartphone o.ä.) wie in FIG. 2B und FIG. 3B beispielhaft dargestellt, oder beispielsweise an eine sonstige externe Datenverarbeitungsvorrichtung, z.B. eine Cloud-Serverarchitektur 226 ("Cloud") wie in FIG. 2A beispielhaft dargestellt, wobei beim Bereitstellen an die Cloud die elektronische Schaltkreisvorrichtung 104 ferner eingerichtet sein kann, ein Ergebnis von der externen Datenverarbeitungsvorrichtung (z.B. der Cloud) 226 zu empfangen und mittels einer Ausgabevorrichtung 128, 228 bereitzustellen.

In verschiedenen Ausführungsbeispielen kann die elektronische Schaltkreisvorrichtung 104 eine Vorrichtung 104a zur kabellosen Datenübertragung aufweisen, beispielsweise für eine Datenübertragung mittels WLAN, Bluetooth, ZigBee, Thread, NFC oder ähnliches, z.B. wie oben beschrieben. Mittels der Vorrichtung 104a zur kabellosen Datenübertragung können Daten an die externe Datenverarbeitungsvorrichtung 226 übertragen oder von dieser empfangen werden.

In verschiedenen Ausführungsbeispielen kann die Haarzustands-Ermittlungsvorrichtung als ein lernendes System gestaltet sein, beispielsweise indem der Nutzer 220 und/oder weitere Nutzer den Haarzustand vor einer Anwendung der Empfehlung und den Haarzustand nach einem Anwenden der Empfehlung der Datenverarbeitungsvorrichtung (beispielsweise mittels der Cloud) bereitstellt/bereitstellen.

In verschiedenen Ausführungsbeispielen kann das Ermitteln des Haarzustands ein computergestütztes Ermitteln des Haarschädigungsgrads und/oder der Haarfarbe oder eines anderen Haarstatus, beispielsweise eines Wassergehalts des Haars aufweisen, beispielsweise mittels prädiktiver Analytik, beispielsweise wie oben beschrieben.

In verschiedenen Ausführungsbeispielen kann das Ermitteln einer Empfehlung ein computergestütztes Ermitteln eines Haarbehandlungsmittels und/oder einer Haarbehandlungsempfehlung, beispielsweise mittels prädiktiver Analytik, unter Einbeziehung des ermittelten Haarzustands aufweisen, beispielsweise wie oben beschrieben.

Im Fall des indirekten Ermittelns kann die externe Datenverarbeitungsvorrichtung 226, z.B. das Smartphone, mit einer entsprechenden Software ausgestattet sein, beispielsweise einer App, beispielsweise wie oben beschrieben.

In verschiedenen Ausführungsbeispielen kann mittels der elektronischen Schaltkreisvorrichtung 104 oder mittels der externen Datenverarbeitungsvorrichtung 226 anhand eines Maßes (z.B. einer Äquivalentbreite) für die (N)IR-Absorption oder der ermittelten Fluoreszenzintensität ein Cysteinsäuregehalt ermittelt werden. Hierfür können, z.B. wie oben beschrieben, mathematische Modelle aus dem Bereich Prädiktive Analytik genutzt werden, um eine Beziehung zwischen dem Maß für die (N)IR-Absorption oder der (standardisierten) Fluoreszenzintensität und einem zugehörigen Cysteinsäuregehalt (und damit einem Schädigungsgrad des Haars) zu ermitteln.

Als unabhängige Parameter können in das Modell dabei in verschiedenen Ausführungsbeispielen beispielsweise eine Beziehung zwischen Cysteinsäuregehalt und (N)IR-Absorption oder Cysteinsäuregehalt und Fluoreszenzintensität oder entsprechende Datenwerte, z.B. in Form zugeordneter Datenpaare, einbezogen werden, welche mittels Vermessung von Standard-Haarproben, welche einen anhand bekannter aufwändiger Verfahren ermittelten Cysteinsäuregehalt aufweisen, ermittelt oder mathematisch modelliert wurden.

Entsprechend kann in verschiedenen Ausführungsbeispielen mit anderen Sensorwerten verfahren werden.

In verschiedenen Ausführungsbeispielen kann der Schädigungsgrad des Haars in einer kategorialen Skala (z.B. leicht, mittel, schwer) ermittelt werden.

In verschiedenen Ausführungsbeispielen kann der Schädigungsgrad in einer metrischen Skala (z.B. Prozentanteil des Gehalts an Cysteinsäure, Lautstärke eines Kämmgeräuschs in dB o.ä.) ermittelt werden.

In verschiedenen Ausführungsbeispielen können anstelle des Prädiktive-Analytik-Verfahrens andere, z.B. einfachere, Verfahren zum Ermitteln des Haarzustands, z.B. des Schädigungsgrads oder der Haarfarbe, und/oder zum Ermitteln der Empfehlung genutzt werden.

Zum Bereitstellen des Haarzustands nach der Behandlung kann in verschiedenen Ausführungsbeispielen ein Ermitteln des Haarzustands nach dem Behandeln mit dem Haarbehandlungsmittel mittels der Haarzustands-Ermittlungsvorrichtung genutzt werden. In anderen Ausführungsbeispielen kann eine andere, beispielsweise baugleiche, Vorrichtung genutzt werden, um den Haarzustand nach der Behandlung zu ermitteln, und der ermittelte Haarzustand kann der Datenverarbeitungsvorrichtung übermittelt werden.

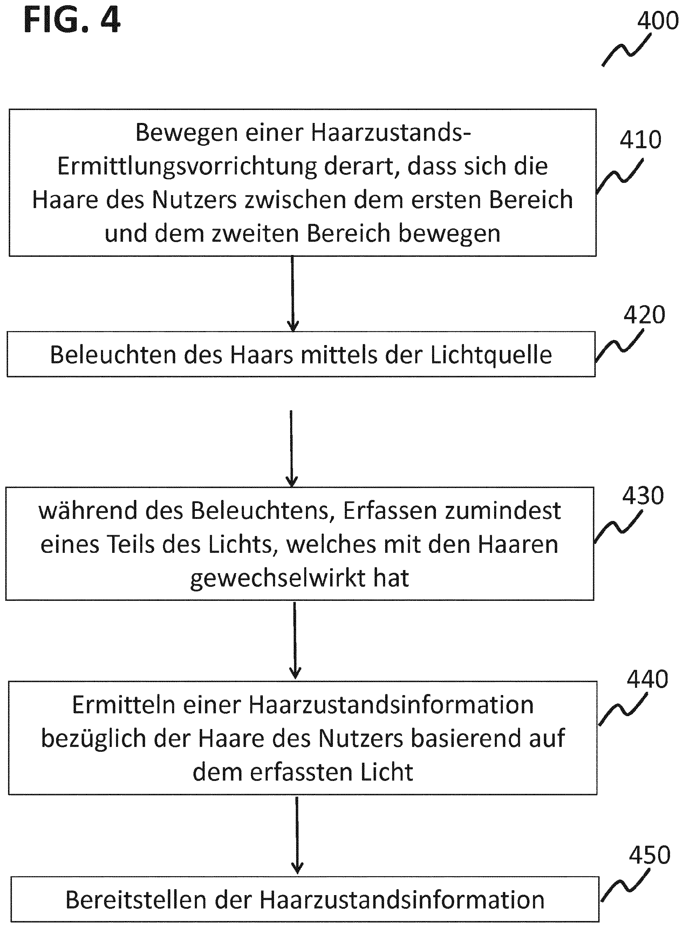

FIG. 4 zeigt ein Ablaufdiagramm 400, welches ein Verfahren zum Bereitstellen eines Haarbehandlungsmittels gemäß verschiedenen Ausführungsbeispielen darstellt. Zum Ausführen des Verfahrens kann eine Vorrichtung gemäß verschiedenen Ausführungsbeispielen wie oben beschrieben verwendet werden.

Das Verfahren kann ein Bewegen einer Haarzustands-Ermittlungsvorrichtung aufweisen, derart, dass sich die Haare des Nutzers zwischen dem ersten Bereich und dem zweiten Bereich bewegen (in 410), ein Beleuchten des Haars mittels der Lichtquelle (in 420), während des Beleuchtens ein Erfassen zumindest eines Teils des Lichts, welches mit den Haaren gewechselwirkt hat (in 430), ein Ermitteln einer Haarzustandsinformation bezüglich der Haare des Nutzers basierend auf dem erfassten Licht (n 440) und ein Bereitstellen der Haarzustandsinformation (in 450).

Weitere vorteilhafte Ausgestaltungen des Verfahrens ergeben sich aus der Beschreibung der Vorrichtung und umgekehrt.

## Patentansprüche

1. Haarzustands-Ermittlungsvorrichtung (100, 100b) zum Bereitstellen einer Information bezüglich eines Haarzustands von Haaren (220H) eines Nutzers (220), aufweisend:
einen Vorrichtungskörper (100K) mit mindestens einem ersten Bereich (100K1) und einem zweiten Bereich (100K2), welche so gestaltet sind, dass die Haare des Nutzers zwischen dem ersten Bereich und dem zweiten Bereich bewegbar sind;
mindestens eine im oder am Vorrichtungskörper angeordnete optionale Lichtquelle (106) zum Beleuchten der Haare des Nutzers;
mindestens einen im oder am Vorrichtungskörper angeordneten optischen Sensor (110) zum Erfassen von Licht, welches von der Lichtquelle abgestrahlt wurde und mit den Haaren gewechselwirkt hat, und/oder von Umgebungslicht, welches mit den Haaren gewechselwirkt hat; und
eine im oder am Vorrichtungskörper angeordnete elektronische Schaltkreisvorrichtung (104),
wobei die elektronische Schaltkreisvorrichtung mit dem optischen Sensor gekoppelt ist zum Empfangen eines mittels des erfassten Lichts erzeugten Signals, und
wobei die elektronische Schaltkreisvorrichtung eingerichtet ist, basierend auf dem empfangenen Signal eine Information bezüglich des Haarzustands des Nutzers zu ermitteln und dem Nutzer die Information bereitzustellen,
**dadurch gekennzeichnet, dass** die Haarzustands-Ermittlungsvorrichtung so eingerichtet ist, dass mittels des optischen Sensors ein Ermitteln eines Grads der Haarschädigung durch eine Bestimmung eines Gehaltes an Cysteinsäure ermittelt wird, wobei der optische Sensor eingerichtet ist, eine oder mehrere Aufnahmen in einem Fluoreszenzbereich und/oder in einem Nahinfrarotbereich zu machen,
wobei die Haarzustands-Ermittlungsvorrichtung ferner so eingerichtet ist, dass mittels des optischen Sensors ein Wassergehalt des Haars ermittelt wird.

2. Haarzustands-Ermittlungsvorrichtung gemäß Anspruch 1,
wobei der Vorrichtungskörper als Kamm oder als Bürste geformt ist, und wobei der erste Bereich und der zweite Bereich zwei benachbarte Kammzinken oder zwei benachbarte Borsten der Bürste aufweisen.

3. Haarzustands-Ermittlungsvorrichtung gemäß einem der Ansprüche 1 oder 2,
wobei die Lichtquelle eine UV-Lichtquelle aufweist und der mindestens eine optische Sensor einen Detektor für sichtbares Licht zum Erfassen von Fluoreszenzlicht aufweist.

4. Haarzustands-Ermittlungsvorrichtung gemäß einem der Ansprüche 1 bis 3,
wobei die Lichtquelle eine NIR-Lichtquelle aufweist und der mindestens eine optische Sensor einen NIR-Detektorzum Erfassen von Nahinfrarotlicht aufweist.

5. Haarzustands-Ermittlungsvorrichtung gemäß einem der Ansprüche 1 bis 4, wobei die elektronische Schaltkreisvorrichtung eingerichtet ist, basierend auf dem ermittelten Haarzustand eine Empfehlung zu ermitteln und dem Nutzer bereitzustellen.

6. Haarzustands-Ermittlungsvorrichtung gemäß Anspruch 5, wobei die Empfehlung mindestens eine aufweist von einer Haarpflegeproduktempfehlung, einer Haarstylingproduktempfehlung, Haarfärbeprodukt und einer Haarbehandlungsempfehlung.

7. Haarzustands-Ermittlungsvorrichtung gemäß einem der Ansprüche 1 bis 6, ferner aufweisend:
mindestens einen weiteren Sensor zum Erfassen eines weiteren Haarzustands.

8. Haarzustands-Ermittlungsvorrichtung gemäß Anspruch 7,
wobei der mindestens eine weitere Sensor ein Mikrofon aufweist.

9. Haarzustands-Ermittlungsvorrichtung gemäß einem der Ansprüche 1 bis 8, wobei die elektronische Schaltkreisvorrichtung eine kabellose Datenaustauschvorrichtung aufweist.

10. Haarzustands-Ermittlungssystem (200), aufweisend:
eine Haarzustands-Ermittlungsvorrichtung gemäß Anspruch 9; und
eine Anzeigevorrichtung (128),
wobei die mindestens eine Haarzustands-Ermittlungsvorrichtung eingerichtet ist, der Anzeigevorrichtung die Haarzustandsinformation mittels der Datenaustauschvorrichtung zu übermitteln.

11. Haarzustands-Ermittlungssystem gemäß Anspruch 10, wobei die Anzeigevorrichtung einen Computerbildschirm, ein Smartphone, ein Tablet oder einen Laptop aufweist.

12. Verfahren zum Bereitstellen einer Haarzustandsinformation bezüglich Haaren (220H) eines Nutzers (220), aufweisend:
Anordnen einer Haarzustands-Ermittlungsvorrichtung (100, 100b) gemäß einem der Ansprüche 1 bis 9 oder eines Haarzustands-Ermittlungssystems (200) gemäß einem der Ansprüche 10 oder 11 derart, dass die Haare des Nutzers einem Lichteintrittsbereich (216) der Haarzustands-Ermittlungsvorrichtung zugewandt sind;
optionales Beleuchten des Haars mittels der Lichtquelle (106);
gegebenenfalls während des Beleuchtens, Erfassen zumindest eines Teils des Lichts, welches mit den Haaren gewechselwirkt hat, und/oder von Umgebungslicht, welches mit den Haaren gewechselwirkt hat;
Ermitteln einer Haarzustandsinformation bezüglich der Haare des Nutzers basierend auf dem erfassten Licht; und
Bereitstellen der Haarzustandsinformation.

13. Verfahren gemäß Anspruch 12, ferner aufweisend:
Ermitteln mindestens einer Empfehlung basierend auf dem ermittelten Haarzustand.

14. Verfahren gemäß Anspruch 13,
wobei die Empfehlung mindestens eine aufweist von einer Haarpflegeproduktempfehlung, Haarfärbeproduktempfehlung, einer Haarstylingproduktempfehlung und einer Haarbehandlungsempfehlung.

15. Verfahren gemäß einem der Ansprüche 12 bis 14,
wobei der ermittelte Haarzustand mindestens einen aufweist von einer Haarschädigung und einem Haarstatus.

## Claims

1. Hair-condition determination device (100, 100b) for providing information regarding a hair condition of hair (220H) of a user (220), comprising:
a device body (100K) having at least a first region (100K1) and a second region (100K2) which are designed so that the user's hair can be moved between the first region and the second region;
at least one optional light source (106) arranged in or on the device body for illuminating the user's hair;
at least one optical sensor (110) arranged in or on the device body for detecting light which has been emitted from the light source and has interacted with the hair and/or ambient light which has interacted with the hair; and
an electronic circuit device (104) arranged in or on the device body,
the electronic circuit device being coupled to the optical sensor for receiving a signal generated by the detected light, and
the electronic circuit device being set up to determine information regarding the hair condition of the user on the basis of the received signal and to provide the information to the user,
**characterized in that**
the hair-condition determination device is set up in such a way that the optical sensor is used to determine a degree of hair damage by determining a cysteic acid content, the optical sensor being set up to take one or more images in a fluorescence range and/or in a near-infrared range,
the hair-condition determination device also being set up in such a way that the water content of the hair is determined by means of the optical sensor.

2. Hair-condition determining device according to claim 1,
wherein the device body is in the form of a comb or a brush, and wherein the first region and the second region comprise two adjacent comb teeth or two adjacent bristles of the brush.

3. Hair-condition determination device according to one of claims 1 or 2,
wherein the light source comprises a UV light source and the at least one optical sensor comprises a visible-light detector for detecting fluorescent light.

4. Hair-condition determination device according to one of claims 1 to 3,
wherein the light source comprises a NIR light source and the at least one optical sensor comprises a NIR detector for detecting near infrared light.

5. Hair-condition determination device according to one of claims 1 to 4,
wherein the electronic circuit device is set up to determine and provide the user with a recommendation on the basis of the determined hair condition.

6. Hair-condition determination device according to claim 5, wherein the recommendation comprises at least one of a hair care product recommendation, a hair styling product recommendation, a hair coloring product and a hair treatment recommendation.

7. Hair-condition determination device according to one of claims 1 to 6, further comprising:
at least one further sensor for detecting a further hair condition.

8. Hair-condition determination device according to claim 7,
wherein the at least one further sensor comprises a microphone.

9. Hair-condition determination device according to one of claims 1 to 8,
wherein the electronic circuit device comprises a wireless data exchange device.

10. Hair-condition determination system (200) comprising:
a hair-condition determination device according to claim 9; and
a display device (128),
wherein the at least one hair-condition determination device is set up to transmit the hair condition information to the display device by means of the data exchange device.

11. Hair-condition determination system according to claim 10,
wherein the display device comprises a computer screen, a smartphone, a tablet or a laptop.

12. Method for providing hair-condition information regarding hair (220H) of a user (220), comprising:
arranging a hair-condition determination device (100, 100b) according to one of claims 1 to 9 or a hair condition determination system (200) according to one of claims 10 or 11 such that the hair of the user faces a light entry region (216) of the hair-condition determination device;
optionally illuminating the hair by means of the light source (106);
optionally during the illuminating, detecting at least a portion of the light that has interacted with the hair and/or ambient light that has interacted with the hair;
determining hair-condition information regarding the user's hair on the basis of the detected light; and
providing the hair-condition information.

13. Method according to claim 12, further comprising:
determining at least one recommendation on the basis of the determined hair condition.

14. Method according to claim 13,
wherein the recommendation comprises at least one of a hair care product recommendation, a hair coloring product recommendation, a hair styling product recommendation, and a hair treatment recommendation.

15. Method according to one of claims 12 to 14,
wherein the determined hair condition comprises at least one of hair damage and hair status.

## Revendications

1. Dispositif de détermination (100, 100b) de l'état des cheveux permettant de fournir des informations concernant un état des cheveux de cheveux (220H) d'un utilisateur (220), comprenant :
un corps de dispositif (100K) comportant au moins une première région (100K1) et une seconde région (100K2), conçu de sorte que les cheveux de l'utilisateur sont mobiles entre la première région et la seconde région ;
au moins une source de lumière (106) facultative disposée dans ou sur le corps de dispositif et permettant d'éclairer les cheveux de l'utilisateur ;
au moins un capteur optique (110) disposé dans ou sur le corps de dispositif et
permettant de saisir de la lumière qui a été émise depuis la source de lumière et qui a interagi avec les cheveux et/ou de la lumière ambiante qui a interagi avec les cheveux ; et
un dispositif de circuit électronique (104) disposé dans ou sur le corps de dispositif, dans lequel le dispositif de circuit électronique est couplé au capteur optique pour recevoir un signal généré au moyen de la lumière saisie, et
dans lequel le dispositif de circuit électronique est configuré pour déterminer des informations concernant l'état des cheveux de l'utilisateur sur la base du signal reçu et pour fournir les informations à l'utilisateur,
**caractérisé en ce que**
le dispositif de détermination de l'état des cheveux est configuré de telle sorte que le capteur optique détermine un degré de l'endommagement des cheveux en déterminant une teneur en acide cystéique, le capteur optique étant configuré pour effectuer un ou plusieurs enregistrements dans une plage de fluorescence et/ou dans une plage du proche infrarouge,
dans lequel le dispositif de détermination de l'état des cheveux est en outre configuré de telle sorte que la teneur en eau des cheveux est déterminée au moyen du capteur optique.

2. Dispositif de détermination de l'état des cheveux selon la revendication 1,
dans lequel le corps de dispositif est en forme de peigne ou de brosse, et dans lequel la première région et la seconde région comprennent deux dents de peigne adjacentes ou deux poils adjacents de la brosse.

3. Dispositif de détermination de l'état des cheveux selon l'une des revendications 1 ou 2, dans lequel la source de lumière comprend une source de lumière UV et l'au moins un capteur optique comprend un détecteur de lumière visible permettant de saisir de la lumière fluorescente.

4. Dispositif de détermination de l'état des cheveux selon l'une des revendications 1 à 3, dans lequel la source de lumière comprend une source de lumière NIR et l'au moins un capteur optique comprend un détecteur NIR permettant de saisir de la lumière infrarouge proche.

5. Dispositif de détermination de l'état des cheveux selon l'une des revendications 1 à 4, dans lequel le dispositif de circuit électronique est configuré pour déterminer et fournir à l'utilisateur une recommandation sur la base de l'état des cheveux déterminé.

6. Dispositif de détermination de l'état des cheveux selon la revendication 5, dans lequel la recommandation comprend une recommandation de produit de soin des cheveux, et/ou une recommandation de produit de coiffage des cheveux, et/ou un produit de coloration des cheveux et/ou une recommandation de traitement des cheveux.

7. Dispositif de détermination de l'état des cheveux selon l'une des revendications 1 à 6, comprenant en outre :
au moins un autre capteur permettant de saisir un autre état des cheveux.

8. Dispositif de détermination de l'état des cheveux selon la revendication 7,
dans lequel l'au moins un autre capteur comprend un microphone.

9. Dispositif de détermination de l'état des cheveux selon l'une des revendications 1 à 8, dans lequel le dispositif de circuit électronique comprend un dispositif d'échange de données sans fil.

10. Système de détermination (200) de l'état des cheveux, comprenant :
un dispositif de détermination de l'état des cheveux selon la revendication 9 ; et
un dispositif d'affichage (128),
dans lequel l'au moins un dispositif de détermination de l'état des cheveux est configuré pour transmettre les informations concernant l'état des cheveux au dispositif d'affichage au moyen du dispositif d'échange de données.

11. Système de détermination de l'état des cheveux selon la revendication 10,
dans lequel le dispositif d'affichage comprend un écran d'ordinateur, un smartphone, une tablette ou un ordinateur portable.

12. Procédé permettant de fournir des informations concernant l'état des cheveux concernant des cheveux (220H) d'un utilisateur (220), comprenant :
la disposition d'un dispositif de détermination (100, 100b) de l'état des cheveux selon l'une des revendications 1 à 9 ou d'un système de détermination (200) de l'état des cheveux selon l'une des revendications 10 ou 11, de telle sorte que les cheveux de l'utilisateur font face à une région d'entrée de lumière (216) du dispositif de détermination de l'état des cheveux ; l'éclairage éventuel des cheveux au moyen de la source de lumière (106) ;
éventuellement lors de l'éclairage, la saisie d'au moins une partie de la lumière ayant interagi avec les cheveux et/ou de la lumière ambiante ayant interagi avec les cheveux ;
la détermination d'informations concernant l'état des cheveux concernant les cheveux de l'utilisateur sur la base de la lumière saisie ; et
la fourniture des informations concernant l'état des cheveux.

13. Procédé selon la revendication 12, comprenant en outre :
la détermination d'au moins une recommandation sur la base de l'état des cheveux déterminé.

14. Procédé selon la revendication 13,
dans lequel la recommandation comprend une recommandation de produit de soin des cheveux, et/ou une recommandation de produit de coloration des cheveux, et/ou une recommandation de produit de coiffage des cheveux et/ou une recommandation de produit de traitement des cheveux.

15. Procédé selon l'une des revendications 12 à 14,
dans lequel l'état des cheveux déterminé comprend au moins l'un parmi un endommagement des cheveux et/ou une condition des cheveux.
